**Europäisches Patentamt**

**European Patent Office**

**Office européen des brevets**

(19)

(11)  **EP 1 433 462 A1**

## (12)  DEMANDE DE BREVET EUROPEEN

(43) Date de publication:
**30.06.2004 Bulletin 2004/27**

(51) Int Cl.$^7$: **A61K 7/021**

(21) Numéro de dépôt: **03293306.1**

(22) Date de dépôt: **23.12.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **24.12.2002 FR 0216654**

(71) Demandeur: **L'OREAL**
**75008 Paris (FR)**

(72) Inventeurs:
- **Camus, Christiane**
 **91170 Viry-Chatillon (FR)**
- **Lee, Hojung**
 **75005 Paris (FR)**

(74) Mandataire: **Tanty, François**
**Nony & Associés,**
**3, rue de Penthièvre**
**75008 Paris (FR)**

(54)  **Compositions de maquillage pour peaux foncées**

(57)  La présente invention concerne une composition de type fond de teint, comportant dans un milieu physiologiquement acceptable, au moins un agent de coloration, caractérisée par le fait qu'elle est apte, lorsqu'elle est appliquée sur une peau foncée, à produire une variation positive de clarté ΔL* comprise entre 0,5 et 4 et une variation positive de saturation ΔC* comprise entre 0,5 et 4.

FIG.3

EP 1 433 462 A1

**Description**

La présente invention concerne les compositions cosmétiques à appliquer sur la peau et plus particulièrement mais non exclusivement les compositions de type fond de teint, destinées aux peaux foncées, y compris noires et métissées.

Par « composition de type fond de teint », on désigne une composition de maquillage de la peau humaine. Il peut s'agir d'un fond de teint à appliquer sur le visage ou le cou, d'un produit anti-cernes, d'un produit correcteur de teint, d'une crème teintée ou base de maquillage pour le visage ou d'une composition de maquillage du corps.

Par « peaux foncées » on désigne des peaux dont la clarté moyenne L* mesurée sur le front, les pommettes et le menton, dans l'espace colorimétrique CIE 1976, est inférieure à 60, notamment à 55. La saturation C* peut être comprise par exemple entre 8 et 30, notamment entre 10 et 30, voire entre 12 et 28. Les valeurs d'angle de teinte h peuvent être comprises par exemple entre 38° environ et 65° environ, notamment entre 46° et 63°, par exemple entre 46° et 54°. Les valeurs de clarté L* peuvent être inférieures ou égales à 50, voire 45 ou 40 pour les peaux les plus sombres, tout en pouvant rester pour la plupart des peaux supérieures à 30. Des peaux foncées sont rencontrées par exemple parmi les populations africaine, afro-américaine, hispano-américaine, indienne et maghrébine.

Il est connu pour éclaircir de telles peaux d'utiliser des produits contenant des actifs blanchissants dont certains sont relativement agressifs comme l'hydroquinone et nécessitent un traitement prolongé pour produire un résultat.

Une autre possibilité est d'utiliser un fond de teint plus clair que sa carnation. Néanmoins, les peaux foncées présentent une grande variété de carnations, ce qui rend difficile la sélection du fond de teint adapté. De plus, le résultat de maquillage peut ne pas apparaître aussi naturel que souhaitable, avec un effet grisâtre et terne notamment.

L'invention vise à proposer une composition cosmétique capable d'éclaircir une peau foncée tout en conférant un maquillage naturel.

L'invention a ainsi pour objet, selon l'un de ses aspects parmi d'autres, une composition de type fond de teint, comprenant dans un milieu physiologiquement acceptable au moins un agent de coloration, la composition pouvant se caractériser par le fait qu'elle est apte, lorsqu'elle est appliquée sur une peau foncée, à produire une variation positive de clarté $\Delta L^*$ comprise entre 0,5 et 4 et une variation positive de saturation $\Delta C^*$ comprise entre 0,5 et 4.

De telles variations de clarté L* et de saturation C* permettent d'éclaircir la peau sans générer d'effet grisâtre ou terne.

Dans des exemples particuliers de mise en oeuvre de l'invention, les variations de clarté $\Delta L^*$ et de saturation $\Delta C^*$ sont comprises entre 0,5 et 3, par exemple entre 0,5 et 2,5. Avec de telles valeurs, le résultat est particulièrement satisfaisant.

La composition peut en outre avantageusement être apte à produire une variation négative d'indice de coxellographie $\Delta i_{cox}$ inférieure ou égale à -0,4, de préférence inférieure ou égale à -0,5, de préférence encore inférieure ou égale à -1, pour une personne présentant avant le maquillage un indice de coxellographie $i_{cox}$ supérieur ou égal à 5. Cela permet d'éclaircir la peau tout en rendant plus homogène la couleur du visage, afin par exemple de dissimuler des zones hyperpigmentées, sans dégrader outre mesure l'aspect naturel de la peau.

Par « *indice de coxellographie* $i_{cox}$ », on désigne le produit des écarts types $\sigma_a^* \times \sigma_b^* \times \sigma_L^*$ pour l'ensemble des zones observées, comme cela sera précisé dans la suite.

Des exemples de compositions éclaircissantes présentent les caractéristiques colorimétriques suivantes :

-   une clarté L* supérieure ou égale à 34, une position b* sur l'axe bleu/jaune comprise entre 28 et 42 et une saturation C* comprise entre 31 et 52, mieux une clarté L* comprise entre 34 et 41, une valeur b* comprise entre 33 et 42 et une saturation C* comprise entre 37 et 46, une telle composition pouvant être utilisée pour éclaircir une peau ayant une carnation définie par une clarté L* inférieure à 42, notamment comprise entre 35 et 42 et une saturation inférieure à 21, notamment comprise entre 13 et 20,
-   une clarté L* supérieure ou égale à 34, une position b* selon l'axe bleu/jaune comprise entre 23 et 39 et une saturation C* comprise entre 27 et 47, mieux une clarté L* comprise entre 34 et 48, une position b* comprise entre 27 et 35 et une saturation C* comprise entre 31 et 41,
-   une clarté L* supérieure ou égale à 34, une position b* sur l'axe bleu/jaune comprise entre 19 et 33 et une saturation C* comprise entre 23 et 40, notamment une clarté L* comprise entre 34 et 42, une position b* comprise entre 23 et 29 et une saturation C* comprise entre 28 et 35,
-   une clarté L* supérieure ou égale à 39, une position b* sur l'axe bleu/jaune comprise entre 23 et 39 et une saturation C* comprise entre 38 et 41, mieux une clarté L* comprise entre 39 et 48, une position b* comprise entre 28 et 35 et une saturation C* comprise entre 33 et 41,
-   une clarté L* supérieure ou égale à 51, une position b* sur l'axe bleu/jaune comprise entre 14 et 25 et une saturation C* comprise entre 18 et 31, mieux une clarté L* comprise entre 51 et 63, une position b* comprise entre 14 et 25 et une saturation C* comprise entre 22 et 28, une telle composition pouvant être utilisée pour éclaircir une peau ayant une clarté comprise entre 48 et 54 et une saturation comprise entre 24 et 28,

- une clarté L* supérieure ou égale à 34, une position b* selon l'axe bleu/jaune comprise entre 11 et 20 et une saturation C* comprise entre 17 et 29, mieux une clarté L* comprise entre 34 et 48, une position b* comprise entre 14 et 17 et une saturation C* comprise entre 20 et 26.

L'invention a encore pour objet, indépendamment ou en combinaison avec ce qui précède, selon un autre de ses aspects, une composition de type fond de teint comprenant, dans un milieu physiologiquement acceptable, au moins un agent de coloration et des particules réfléchissantes, ladite composition possédant une teinte comprise dans la gamme de couleurs s'étendant du beige rosé au brun orangé.

L'invention a encore pour objet, indépendamment ou en combinaison avec ce qui précède, selon un autre de ses aspects, une composition de type fond de teint, comprenant, dans un milieu physiologiquement acceptable, au moins un agent de coloration et des particules réfléchissantes, ladite composition possédant un angle de teinte h variant de 40° à 70°, notamment 50° à 70°, et une saturation C* variant de 20 à 50.

L'invention a encore pour objet, indépendamment ou en combinaison avec ce qui précède, selon un autre de ses aspects, une composition de type fond de teint, comprenant, dans un milieu physiologiquement acceptable, au moins un agent de coloration et des particules réfléchissantes, ladite composition ayant une réflectance variant de 10 à 45 % dans l'intervalle 600 à 680 nm.

L'invention a encore pour objet, indépendamment ou en combinaison avec ce qui précède, selon un autre de ses aspects, un produit de type fond de teint notamment pour le maquillage de la peau, en particulier des peaux foncées, comprenant au moins une première et un seconde compositions, chacune dans un contenant, la première composition comprenant, dans un premier milieu physiologiquement acceptable, au moins un agent de coloration et la seconde composition comprenant, dans un second milieu physiologiquement acceptable, au moins des particules réfléchissantes.

La présente invention a également pour objet, selon un autre de ses aspects, un procédé de maquillage d'une peau foncée, comprenant l'application sur la peau d'un produit à deux compositions tel que défini ci-dessus. Ce procédé peut comprendre l'application d'une première couche de l'une des deux compositions, dite composition de base puis l'application sur au moins une partie de la première couche d'une deuxième couche de l'autre composition, dite composition de surface.

Le maquillage ainsi obtenu est un maquillage bicouche. L'ordre de superposition et/ou le mode de superposition des deux compositions, à savoir total ou partiel, peuvent par ailleurs conférer le cas échéant des effets esthétiques supplémentaires.

L'invention a encore pour objet, selon un autre de ses aspects, l'utilisation de l'une des compositions telles que définies ci-dessus pour éclaircir une peau foncée.

L'invention a encore pour objet, selon un autre de ses aspects, un procédé pour commercialiser une composition destinée à éclaircir une peau foncée, qui comporte l'étape consistant à faire état, lors de la commercialisation de cette composition, de la couleur des peaux auxquelles la composition est préférentiellement destinée. Un tel procédé peut comporter la présentation, par tout moyen, des couleurs de peaux auxquelles la composition est destinée, par exemple grâce à l'utilisation d'un témoin coloré figurant sur un récipient ou un emballage contenant la composition. Il peut encore être fait référence sur l'emballage ou le récipient à une ethnie dans laquelle on retrouve fréquemment le type de couleur de peau auquel la composition est destinée.

L'invention a encore pour objet un procédé dans lequel on mesure une caractéristique colorimétrique d'une peau foncée, notamment sa clarté et/ou sa saturation, et dans lequel on sélectionne à partir de la caractéristique colorimétrique mesurée une composition adaptée à éclaircir cette peau, notamment une composition produisant des variations de clarté et de saturation, et éventuellement d'indice de coxellographie, telles que mentionnées plus haut.

Indépendamment ou en combinaison avec ce qui précède, l'invention a encore pour objet une composition de type fond de teint, comprenant dans un milieu physiologiquement acceptable, au moins un agent de coloration, cette composition pouvant se caractériser par le fait qu'elle est apte à présenter un pouvoir d'homogénéisation $1/\Delta E_1$ et un pouvoir couvrant $1/\Delta E_2$ suivants, lorsqu'elle est appliquée, suivant la valeur de sa clarté L*, sur l'une des cartes de contraste suivantes :

- lorsque la composition a une clarté L* comprise entre 30 et 40, qu'elle est appliquée sur une carte de contraste présentant cinq zones ayant respectivement pour coordonnées colorimétriques, à 15 % près, voire à 12,5 %, mieux à 10 % près, voire à 7,5 % près, voire à 5 % près, pour L* et h, et à 25 % près, mieux à 20 %, encore mieux 15 %, voire à 10 %, ou voire 5 % près, pour C*,

  - première zone (B11+): L* = 36,7 C* = 19,81 h = 47,34°,
  - deuxième zone (B11) : L* = 38,43 C* = 21,76 h = 46,51°,
  - troisième zone (B12): L* = 35,66 C* = 19,78 h = 46,32°,
  - quatrième zone (B12+): L* = 32,98 C* = 17,29 h = 44,64°,

- cinquième zone (XXX): L* = 29,63 C* = 15,06 h = 40,34°,

la composition est apte à présenter un pouvoir d'homogénéisation $1/\Delta E_{1\ moyen}$ compris entre environ 1,1 et environ 6,7, mieux entre environ 1,25 et environ 5, et un pouvoir couvrant $1/\Delta E_2$ compris entre environ 0,25 et environ 0,84.

- lorsque la composition a une clarté comprise entre 40 et 50, qu'elle est appliquée sur une carte de contraste présentant cinq zones ayant respectivement pour coordonnées colorimétriques, à 15 % près, mieux à 12,5 %, mieux encore à 10 %, voire à 7,5 % près, voire à 5 % près pour L* et h, et à 25 % près, mieux à 20 %, encore mieux à 15 %, voire 10 %, ou 5 % près, pour C*,

  - première zone (C9): L* = 45,04 C* = 25,18 h = 53,27°,
  - deuxième zone (B12): L* = 35,66 C* = 19,78 h = 46,32°,
  - troisième zone (C11): L* = 38,73 C* = 21,94 h = 50,18°,
  - quatrième zone (C10): L* = 42,19 C* = 24,18 h = 51,94°,
  - cinquième zone (C8): L* = 48,06 C* = 25,97 h = 53,09°,

la composition est apte à présenter un pouvoir d'homogénéisation $1/\Delta E_{1\ moyen}$ compris entre environ 1,1 et environ 1,54, mieux entre environ 1,1 et environ 1,43, et un pouvoir couvrant $1/\Delta E_2$ compris entre environ 1/9 et 1/5, mieux entre environ 1/8 et environ 0,15,

- lorsque la composition présente une clarté comprise entre 50 et 60, qu'elle est appliquée sur une carte de contraste présentant cinq zones ayant respectivement pour coordonnées colorimétriques, à 15 % près, mieux à 12,5 %, mieux encore à 10 %, voire à 7,5 % près, voire à 5 % près pour L* et h, et à 25 % près, mieux à 20 %, encore mieux à 15 %, voire 10 ou 5 % près, pour C*,

  - première zone (D6) : L* = 54,08 C* = 26,70 h = 57,35°,
  - deuxième zone (C11) : L* = 38,73 C* = 21,94 h = 50,18°,
  - troisième zone (D8) : L* = 47,94 C* = 26,18 h = 56,82°,
  - quatrième zone (D7) : L* = 51,79 C* = 27,21 h = 57,09°,
  - cinquième zone (D5): L* = 57,61 C* = 26,22 h = 55,09°,

la composition est apte à présenter un pouvoir d'homogénéisation $1/\Delta E_{1\ moyen}$ compris entre environ 0,8 et environ 1,25, et un pouvoir couvrant $1/\Delta E_2$ compris entre environ 1/7 et 1/3, les pouvoirs d'homogénéisation et couvrant étant définis plus loin.

Indépendamment ou en combinaison avec ce qui précède, l'invention a encore pour objet une composition de type fond de teint comprenant, dans un milieu physiologiquement acceptable, au moins un agent de coloration, cette composition pouvant se caractériser par le fait qu'elle est apte à présenter, lorsqu'elle est appliquée sur une carte de contraste présentant cinq zones ayant respectivement pour coordonnées colorimétriques, à 5 % près :

- première zone (Z1): L* = 48,38 a* = 7,99 b* = 3,85
- deuxième zone (Z2) : L* = 46,67 a* = 6,78 b* = 3,25
- troisième zone (Z3) : L* = 44, 5 a* = 6,76 b* = 3,1
- quatrième zone (Z4) : L* = 42,72 a* = 4,12 b* = 2,57
- cinquième zone (Z5): L* = 44,41 a* = 6,57 b* = 3,93

et une sixième zone (Z6) ayant pour coordonnées colorimétriques
      L* = 52,26       a* = 9,11       b* = 5,81,
un pouvoir d'homogénéisation $1/\Delta E_{1moyen}$ compris entre 1/4 et 1, mieux entre 1/3 et 1/2, et un pouvoir couvrant $1/\Delta E_2$ compris entre 1/25 et 1/7, mieux entre 1/21 et 1/10, les pouvoirs d'homogénéisation et couvrant étant définis plus loin.
Des compositions présentant des effets éclaircissants adaptés à certaines carnations au moins peuvent présenter par exemple, lorsqu'elles sont appliquées sur la carte de contraste comportant les six zones (Z1, Z2, Z3, Z4, Z5 et Z6) précitée :

- un pouvoir d'homogénéisation $1/\Delta E_{1\ moyen}$ compris entre 1/1,6 et 1/2 et un pouvoir couvrant $1/\Delta E_2$ compris entre 1/12 et 1/15,
- un pouvoir d'homogénéisation $1/\Delta E_{1\ moyen}$ compris entre 1/1,8 et 1/2, et un pouvoir couvrant $1/\Delta E_2$ compris entre 1/13 et 1/17,
- un pouvoir d'homogénéisation $1/\Delta E_{1\ moyen}$ compris entre 1/1,6 et 1/2,1 et un pouvoir couvrant $1/\Delta E_2$ compris entre 1/12 et 1/16,

- un pouvoir d'homogénéisation $1/\Delta E_{1\,moyen}$ compris entre 1/2,6 et 1/3 et un pouvoir couvrant $1/\Delta E_2$ compris entre 1/16 et 1/21, ou
- un pouvoir d'homogénéisation $1/\Delta E_{1\,moyen}$ compris entre 1/1,7 et 1/2, et un pouvoir couvrant $1/\Delta E_2$ compris entre 1/9 et 1/13.

L'invention a encore pour objet, indépendamment ou en combinaison avec ce qui précède, une carte de contraste pouvant se caractériser par le fait qu'elle comporte au moins deux zones colorées correspondant respectivement à la couleur moyenne d'au moins deux régions du visage d'un panel d'individus.

La carte peut comporter également une autre zone colorée correspondant à la couleur moyenne d'une région du corps située ailleurs que sur le visage.

Toutes les zones colorées peuvent avantageusement être réalisées de manière à présenter sensiblement la même couleur sous deux illuminants distincts.

Dans une réalisation particulière, la carte comporte au moins trois zones colorées correspondant respectivement à la couleur moyenne du front, d'une poche sous les yeux et de la région située entre la lèvre supérieure et le nez des individus du panel. La carte peut comporter en outre deux zones colorées correspondant à la couleur de taches de peau d'individus du panel.

La carte peut encore comporter une zone blanche, voire également une zone noire.

L'invention a encore pour objet, selon un autre de ses aspects, un procédé pour déterminer au moins une caractéristique colorimétrique d'une composition, comportant les étapes suivantes :

- recouvrir une carte de contraste parmi celles qui sont définies ci-dessus d'une couche d'une composition,
- mesurer la couleur desdites zones de la carte au travers de la composition,
- déterminer au moins une caractéristique colorimétrique de la composition, notamment le pouvoir d'homogénéisation et/ou le pouvoir couvrant, en fonction d'écarts de couleur mesurés entre lesdites zones.

La composition, notamment lorsqu'elle est liquide, peut être appliquée sur un support transparent déposé sur la carte, ce qui permet de ne pas altérer celle-ci. Un tel support peut avantageusement être adhésif lorsque la composition est pulvérulente.

L'invention a encore pour objet, selon un autre de ses aspects, un procédé pour fabriquer une carte de contraste permettant d'évaluer au moins une caractéristique colorimétrique d'une composition, comportant les étapes suivantes :

- sélectionner un panel d'individus ayant une même typologie de peau :

    - pour chaque individu du panel,

        ■ mesurer la couleur d'une région au moins du corps située ailleurs que sur le visage,
        ■ mesurer la couleur d'au moins une région du visage,

- calculer une couleur moyenne pour chaque région,
- reproduire par impression les couleurs moyennes ainsi calculées sur une carte de contraste.

L'invention a encore pour objet, selon un autre de ses aspects, un procédé pour fabriquer une carte de contraste permettant d'évaluer au moins une caractéristique colorimétrique d'une composition, comportant les étapes suivantes :

- sélectionner un panel d'individus ayant une même typologie de peau :

    - pour chaque individu du panel,

        ■ mesurer la couleur d'au moins deux régions différentes du visage,

- calculer une couleur moyenne pour chaque région,
- reproduire par impression les couleurs moyennes ainsi calculées sur une carte de contraste.

Pour un individu au moins du panel, et de préférence pour tous les individus du panel, on peut mesurer la couleur d'au moins trois régions différentes du visage, notamment le front, la région entre les lèvres et le nez et les cernes sous les yeux.

L'invention a encore pour objet, selon un autre de ses aspects, un procédé de fabrication d'une composition à appliquer sur une peau ayant une typologie donnée, comportant les étapes suivantes :

- sélectionner au moins un agent de coloration de la composition en utilisant une carte de contraste telle que définie ci-dessus,
- fabriquer la composition avec cet agent de coloration.

L'invention pourra être mieux comprise à la lecture de la description détaillée qui va suivre, d'exemples de mise en oeuvre non limitatifs de celle-ci, et à l'examen du dessin annexé, sur lequel :

- la figure 1 représente de manière schématique et partielle un dispositif d'acquisition pouvant être utilisé pour effectuer des mesures colorimétriques,
- les figures 2 et 3 représentent de manière schématique un visage placé dans l'ouverture de la sphère du dispositif de la figure 1, sur lequel on effectue des mesures colorimétriques,
- la figure 4 représente des exemples de cartes de contraste pouvant être utilisées pour déterminer des caractéristiques colorimétriques de compositions destinées à éclaircir la peau,
- les figures 5 et 6 représentent d'autres exemples de cartes de contraste pouvant être utilisées pour déterminer des caractéristiques colorimétriques de compositions destinées à éclaircir la peau,
- la figure 7 représente des spectres de réflectance de compositions,
- la figure 8 représente de manière schématique un exemple de conditionnement comportant au moins un témoin coloré, et
- le tableau I représente des exemples de valeurs de réflectance spectrale en pourcentages en fonction de la longueur d'onde et des zones.

## Mesures de L* et C* (dans l'espace CIE 1976)

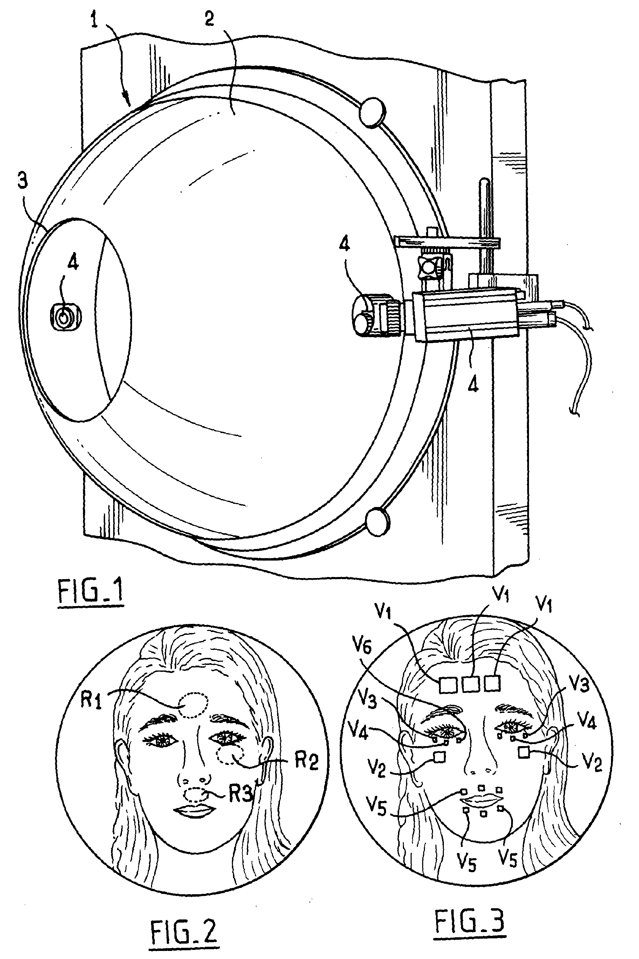

La clarté L* et la saturation C* peuvent être mesurées avant et après le maquillage au moyen d'un dispositif d'acquisition 1 que l'on a représenté schématiquement et partiellement à la figure 1, comportant une sphère 2 à l'intérieur de laquelle sont disposées plusieurs sources de lumière blanche, non apparentes, de manière à ce que la lumière gagnant son ouverture 3 soit la plus homogène possible.

Le visage de la personne dont on souhaite mesurer la couleur est placé dans cette ouverture 3 et des images sont acquises au moyen de caméras couleur 4 ou d'appareils de photographie numérique.

Préalablement à l'acquisition des images, la chaîne d'acquisition est calibrée en plaçant dans le champ des caméras des étalons colorimétriques de référence, par exemple selon le procédé décrit dans le brevet US 6 362 849, dont le contenu est incorporé à la présente demande par référence.

Les mesures de la couleur peuvent être effectuées par exemple sur trois régions du visage, à savoir dans l'exemple de la figure 2 une première région R1 située sur le front, une deuxième région R2 correspondant aux cernes sous les yeux et une troisième région R3 entre le nez et les lèvres.

La couleur est mesurée avant le maquillage sur la peau préalablement nettoyée puis après le maquillage, par exemple 15 min après la fin de celui-ci.

Les compositions de maquillage sont dans l'exemple considéré des fonds de teint liquides appliqués de manière usuelle directement sur la peau, sans dépôt d'une base au préalable, à raison de 0,5 à 1 mg environ par $cm^2$, de préférence entre 0,7 et 0,8 $mg/cm^2$. Bien entendu, l'invention n'est pas limitée à des compositions se présentant sous la forme de liquides, et s'applique également à des compositions se présentant sous la forme de sticks ou de poudres libres ou compactées. L'invention s'applique également à des compositions distribuées sous forme de spray.

Chaque mesure est effectuée sur une image sur laquelle apparaissent les zones dont on cherche à déterminer la couleur, les régions R1, R2 ou R3 correspondant chacune par exemple à plus de 100 pixels de l'image, par exemple environ 250 pixels. Pour chaque pixel, les coordonnées L*, a* et b* sont déterminées avant et après le maquillage.

## Calcul de l'indice de coxellographie $i_{cox}$

On peut calculer l'écart type σ pour chaque paramètre L*, a* et b* pour l'ensemble des pixels des trois régions R1, R2 et R3, c'est-à-dire par exemple pour environ 750 pixels, avant et après maquillage.

L'indice de coxellographie $i_{cox}$ est défini comme étant le produit des trois écarts types $\sigma_a^*$, $\sigma_b^*$ et $\sigma_L^*$ : $i_{cox} = \sigma_a^* \times \sigma_b^* \times \sigma_L^*$

Plus la couleur de la peau est homogène, plus l'indice de coxellographie $i_{cox}$ est faible.

En comparant les clarté L* et saturation C* avant et après le maquillage des régions R1, R2 et R3, le maquillage a pu être considéré comme étant satisfaisant lorsque les variations ΔL* de la clarté et ΔC* de la saturation étaient comprises entre 0,5 et 4, mieux entre 0,5 et 2,5.

Pour des personnes dont la peau est inhomogène et présente un indice de coxellographie $i_{cox} \geq 5$, un effet d'homogénéisation a pu être retenu lorsque l'application du maquillage entraînait une variation de l'indice de coxellographie $i_{cox} \leq -0,4$.

**Cartes de contraste**

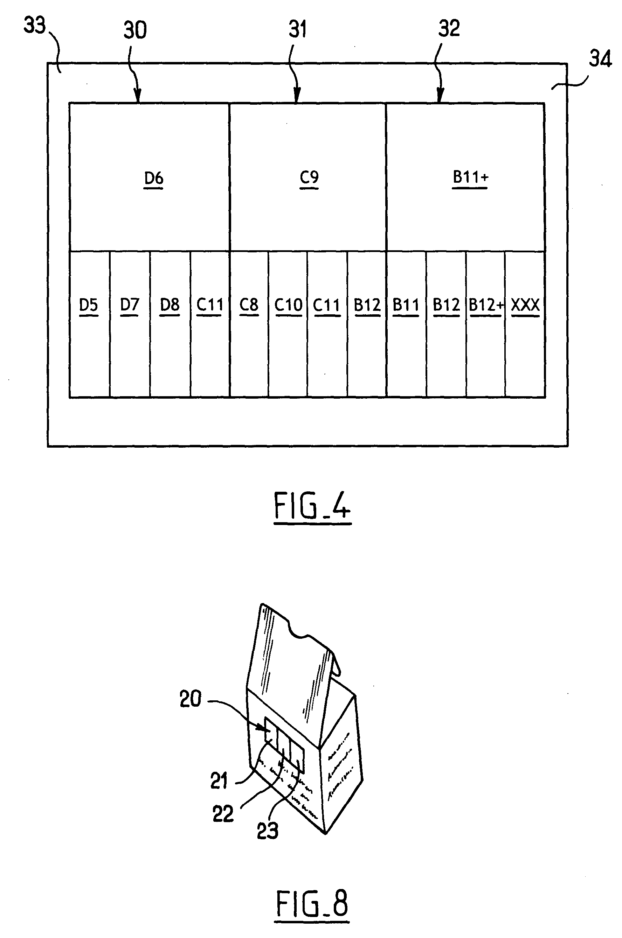

On peut avantageusement utiliser des cartes de contraste, comme illustré sur la figure 4, pour mettre en évidence notamment les pouvoirs d'homogénéisation et couvrant d'une composition.

Sur cette figure, on a représenté trois cartes de contraste 30, 31 et 32 réunies sur un même support 33. On ne sort pas du cadre de la présente invention si les cartes de contraste 30, 31 et 32 sont réalisées sur des supports distincts.

Le support 33 comporte une bordure blanche 34 s'étendant tout autour des cartes de contraste 30, 31 et 32 qui sont accolées.

La carte de contraste 30 comporte une zone colorée D6, par exemple carrée, et quatre zones colorées D5, D7, D8 et C11, par exemple rectangulaires, disposées côte à côte et accolées à la zone colorée D6 par un de leurs petits côtés.

La carte de contraste 31 comporte une zone colorée carrée C9, et quatre zones colorées rectangulaires C8, C10, C11 et B12.

De même, la carte de contraste 32 comporte une zone colorée carrée B11+ et quatre zones colorées rectangulaires B11, B12, B 12+ et XXX.

Les différentes zones D5 à D8, C8 à C11, B11, B11+, B12, B12+ et XXX sont de préférence réalisées par impression d'encres choisies de manière à ce que ces zones apparaissent pour un observateur de la même couleur sous au moins deux illuminants différents, par exemple sous au moins deux des illuminants D65, D50 et A. On pourra à ce sujet utilement se référer à la demande de brevet européen EP 1 212 961 dont le contenu est incorporé à la présente par référence.

De préférence, les réflectances spectrales pour au moins certaines voire la totalité des zones ci-dessus sont à 25 % près, mieux à 20 % près, mieux encore à 15 % près, mieux encore à 10 % près, mieux encore à 5 % près celles données dans le tableau I, pour au moins l'une des longueurs d'onde indiquées dans le tableau et de préférence la totalité des longueurs d'onde données dans celui-ci.

Les réflectances spectrales sont mesurées avec un spectrocolorimètre de marque MINOLTA®, de référence CM2002, en mode réflexion, spéculaire inclus, moyenne ouverture.

Le support 33 est par exemple une feuille de papier de 130 g/cm$^2$, mat, pour impression jet d'encre.

La carte de contraste 30 peut être représentative des peaux dont la clarté est comprise entre 50 et 60.

Les différentes zones D6, D5, D7, D8 et C11 de la carte de contraste 30 peuvent ainsi correspondre à des couleurs moyennes observées sur un panel d'individus ayant une peau foncée de carnation claire pour différentes régions du visage.

Par exemple, la zone D6 peut correspondre à une moyenne des couleurs observées sur le front et les pommettes, la couleur étant mesurée sur le front dans des zones V1 de l'image du visage comme on peut le voir sur la figure 3 et sur les pommettes dans les zones V2. La zone D5 correspond par exemple à la couleur des pommettes, mesurée dans la zone V2 de l'image, la zone D7 par exemple à la couleur du front dans les zones V1, la zone D8 peut correspondre à une moyenne de la couleur des cernes externes mesurée dans la zone V3, de celle des cernes du milieu mesurée dans la zone V4 et de celle mesurée autour des lèvres dans les zones V5. La zone C11 peut correspondre à la couleur mesurée au niveau du cerne interne dans la zone V6.

La carte de contraste 31 peut être représentative des peaux dont la clarté est comprise entre 40 et 50.

Ainsi, les différentes zones C9, C8, C10, C11 et B12 de la carte de contraste 31 peuvent correspondre à des couleurs moyennes observées sur un panel d'individus ayant une peau foncée de carnation moyenne pour différentes régions du visage.

La zone C9 correspond par exemple à la couleur mesurée sur le front, dans les zones V1 de l'image moyennée avec la couleur mesurée sur les pommettes, dans les zones V2.

La zone C8 peut correspondre à la couleur mesurée sur les pommettes, dans la zone V2 de l'image. La zone C10 peut correspondre à la couleur mesurée sur le front, dans les zones V1.

La zone C11 correspond par exemple à la couleur moyenne entre celle des cernes externes, mesurée dans la zone V3, celle des cernes du milieu, mesurée dans la zone V4, et celle de la région autour des lèvres, dans la zone V5. La région B12 peut correspondre à la couleur des cernes internes, mesurée dans la région V6.

La carte de contraste 32 peut être représentative des peaux dont la clarté est comprise entre 30 et 40.

Ainsi, les différentes zones B11+, B11, B12, B12+ et XXX peuvent correspondre à des couleurs moyennes observées sur un panel d'individus ayant une peau foncée de carnation sombre pour différentes régions du visage.

La région B11+ peut correspondre à la moyenne de la couleur mesurée sur le front dans les zones V1 moyennée avec celle mesurée sur les pommettes, dans les zones V2.

La zone B11 peut correspondre à la couleur mesurée sur les pommettes, dans la zone V2, la zone B12 à celle mesurée sur le front, dans les zones V1, la zone B12+ à la couleur moyenne entre celle mesurée sur les cernes externes dans les zones V3, celle mesurée dans les cernes du milieu dans les zones V4 et celle mesurée autour des lèvres dans les zones V5. La zone XXX peut correspondre à la couleur des cernes internes, mesurée dans les zones V6.

On a réalisé une carte de contraste 32 sur laquelle on a pu mesurer, pour chaque zone, les valeurs suivantes :

| Zone | B11+ | XXX | B12+ | B12 | B11 |
|------|------|------|------|------|------|
| L* | 36,7 | 29,63 | 32,98 | 35,66 | 38,43 |
| C* | 19,81 | 15,06 | 17,29 | 19,78 | 21,76 |
| h | 47,34° | 40,34° | 44,64° | 46,32° | 46,51° |

On a réalisé une carte de contraste 31 sur laquelle on a pu mesurer, pour chaque zone, les valeurs suivantes :

| Zone | C9 | B12 | C11 | C10 | C8 |
|------|------|------|------|------|------|
| L* | 45,04 | 35,66 | 38,73 | 42,19 | 48,06 |
| C* | 25,18 | 19,78 | 21,94 | 24,18 | 25,97 |
| h | 53,27° | 46,32° | 50,18° | 51,94° | 53,09° |

On a réalisé une carte de contraste 30 sur laquelle on a pu mesurer, pour chaque zone, les valeurs suivantes :

| Zone | D6 | C11 | D8 | D7 | D5 |
|------|------|------|------|------|------|
| L* | 54,08 | 38,73 | 47,94 | 51,79 | 57,61 |
| C* | 26,70 | 21,94 | 26,18 | 27,21 | 26,22 |
| h | 57,35° | 50,18° | 56,82° | 57,09° | 55,09° |

Les mesures ont été effectuées avec un spectrocolorimètre de marque MINOLTA®, de référence CM2002, en mode réflexion, spéculaire inclus, moyenne ouverture.

Les réflectances spectrales mesurées sont celles données dans le tableau I, ces valeurs étant données à titre d'exemple non limitatif uniquement.

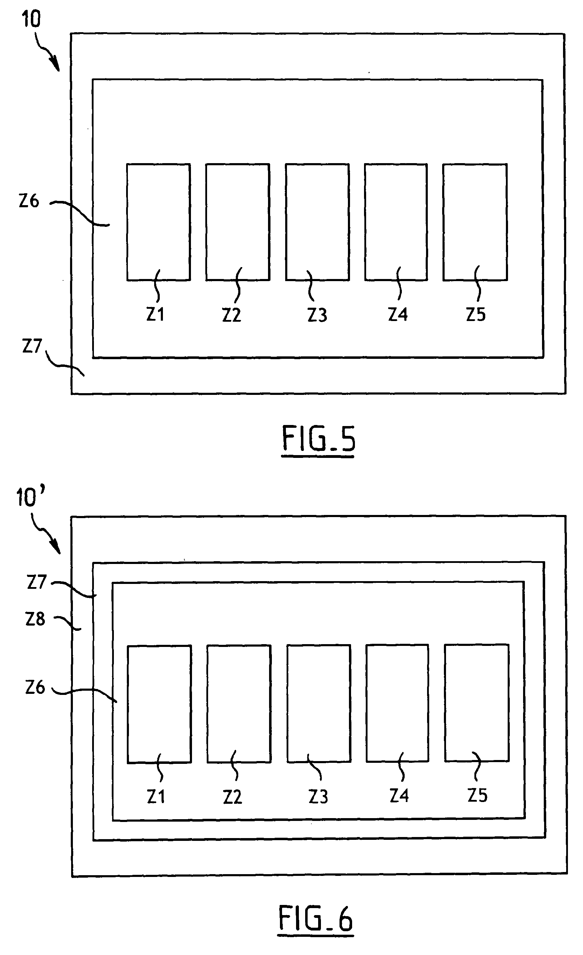

Sur les figures 5 et 6, on a représenté d'autres exemples de cartes de contraste 10 et 10' comportant chacune cinq zones colorées Z1, Z2, Z3, Z4 et Z5, par exemple rectangulaires, disposées côte à côte au centre d'une sixième zone Z6 occupant dans les exemples considérés une bonne partie du fond de la carte.

Les cartes 10 et 10' comportent également une bordure blanche Z7 s'étendant tout autour de la zone Z6 à la manière d'un cadre et la carte 10' diffère de la carte 10 seulement par le fait qu'elle comporte en outre une zone Z8 noire s'étendant autour de la zone Z7 blanche. Cette zone noire Z8 peut permettre d'évaluer avec la zone blanche Z7 la couvrance de la composition d'une manière similaire à ce que permettent les cartes de contraste usuelles noir/blanc.

Les différentes zones Z1 à Z7, et Z8 éventuellement, sont de préférence réalisées par impression d'encres choisies de manière à ce que ces zones apparaissent pour un observateur de la même couleur sous au moins deux illuminants différents, par exemple sous au moins deux des illuminants D65, D50 et A.

Les différentes zones Z1 à Z6 peuvent correspondre à des couleurs moyennes observées sur un panel d'individus ayant une peau foncée, par exemple une peau appartenant à un type donné de peau noire, pour différentes régions du visage et du corps. On peut par exemple considérer comme étant d'un même type les peaux dont la clarté est comprise entre 45 et 50 (peaux foncées claires), entre 39 et 44 (peaux foncées moyennes), et inférieure à 39 (peaux foncées sombres).

Les zones Z1 à Z5 peuvent par exemple correspondre respectivement à la couleur moyenne mesurée sur le front, les poches sous les yeux, l'espace entre le nez et les lèvres, ainsi que sur des taches du visage, notamment des taches présentes sur les joues, et la zone Z6 à la couleur mesurée sur le corps, par exemple le dessous de l'avant-bras.

Dans l'exemple considéré, les zones Z1, Z2, Z3 de la carte 10 correspondent respectivement à la couleur des régions R1, R2 et R3 représentées à la figure 2.

On a réalisé une carte 10 sur laquelle on a pu mesurer pour chaque zone les valeurs suivantes :

| Zone | Z1 | Z2 | Z3 | Z4 | Z5 | Z6 |
|------|------|------|------|------|------|------|
| L* | 48,38 | 46,67 | 44,5 | 42,72 | 44,41 | 52,26 |
| a* | 7,99 | 6,78 | 6,76 | 4,12 | 6,57 | 9,11 |
| b* | 3,85 | 3,25 | 3,1 | 2,57 | 3,93 | 5,81 |

Les mesures ont été effectuées avec un spectrocolorimètre de marque MINOLTA®, de référence CM3700d, en mode réflexion, spéculaire inclus, UV inclus, petite ouverture d/8.

Pour toutes les cartes de contraste, les valeurs de L* et h sont données à 15 % près, voire à 12,5 % près, mieux à 10 % près, voire à 7,5 % près, voire à 5 % près et les valeurs de C* sont données à 25 % près, mieux à 20 % près, encore mieux à 15 % près, voire à 10 % ou voire 5 % près.

## Formulations

On va maintenant décrire dans la suite des exemples de composés permettant de réaliser des compositions éclaircissantes, lesquelles comportent au moins un agent de coloration, associé de préférence à des particules réfléchissantes. Les compositions éclaircissantes peuvent comporter également des charges. Le milieu physiologiquement acceptable peut comporter une phase aqueuse ou une phase grasse. La composition peut encore comporter au moins un agent tensioactif et/ou au moins un polymère filmogène.

### Agents de coloration

Des agents de coloration convenant à l'invention peuvent produire, seuls ou en mélange, une coloration jaune ou orangée. En d'autres termes, ils possèdent une réflectance significative dans l'intervalle 550 à 675 nm.

Le ou les agents de coloration peuvent être présents dans la composition de type fond de teint, notamment la composition de base ou de surface du produit de type fond de teint selon l'invention, à une teneur allant de 0,5 à 30 % en poids, notamment allant de 2 % à 20 % en poids et en particulier de 5 à 18 % en poids par rapport au poids total de la composition considérée.

Le ou les agents de coloration, peuvent être choisis parmi les pigments minéraux ou organiques, les polymères colorants, les colorants hydrosolubles ou liposolubles, les laques organiques, les poudres métalliques et leurs mélanges. Ils peuvent notamment être choisis parmi ceux cités dans le C.T.F.A Cosmetic Ingredient Handbook, 3ième Edition Cosmetic and fragrance Assn., Inc., Washington D.C. (1982).

A titre illustratif et non limitatif des agents de coloration minéraux, on peut plus particulièrement citer les oxydes métalliques jaunes, rouges, bruns comme par exemple les oxydes de fer.

Comme poudres métalliques, on peut citer la poudre de cuivre.

Conviennent notamment comme pigments organiques, les pigments FDC Yellow n° 5 (sel disodique de tartrazine).

A titre illustratif des laques organiques convenant à l'invention, on peut plus particulièrement citer les FDC Yellow n° 5 et n° 6 A1 Laque.

Les colorants hydrosolubles peuvent être choisis par exemple parmi le colorant brun identifié par l'appellation « caramel » selon le Color Index ; les colorants jaunes identifiés par les numéros de Color Index n° 10316, 13015, 18690, 18820, 18965, 19140, 45430, 47005, 75100 et celui dénommé Lactoflavine ; les colorants oranges identifiés par les numéros de Color Index n° 14270, 15510, 15980, 15985, 16230, 20170, 40215 ; les colorants rouges identifiés par les numéros de Color Index n° 14700, 14720, 14815, 15620, 16035, 16185, 16255, 16290, 17200, 18050, 18130, 18736, 24790, 27290, 45100, 45220, 45380, 45405, 45410, 45425, 45430, 75470 et leurs mélanges.

Les colorants liposolubles peuvent être choisis par exemple parmi le colorant brun identifié par le numéro Color Index n° 12010 ; les colorants jaunes identifiés respectivement par les numéros de Color Index n° 12700, 21230, 47000, 75125, 75135 ; les colorants oranges identifiés par les numéros de Color Index n° 11920, 40800, 40820, 40825, 40850, 45396, 75120, 75130 et la capasanthine et le colorant rouge identifié par le n° 12150 et leurs mélanges.

Le polymère colorant est un polymère comportant au moins un groupement colorant organique. Le groupement colorant peut être greffé, notamment par liaison covalente, sur la chaîne du polymère. Le polymère colorant contient en général moins de 10 % en poids, par rapport au poids total du polymère, de matière colorante.

Ce polymère colorant peut être de toute nature chimique, notamment polyester, polyamide, polyuréthanne, polyacrylique, poly(méth)acrylique, polycarbonate, d'origine naturelle comme les polymères cellulosiques ou de chitosane, ou un de leurs mélanges, et en particulier un polyester ou polyuréthanne.

En particulier, le polymère colorant peut être un copolymère à base d'au moins deux monomères distincts dont l'un au moins est un monomère colorant organique.

De tels polymères colorants sont notamment décrits dans les brevets ou demandes de brevet US 5 032 670, US 4 999 418, US 5 106 942, US 5 030 708, US 5 102 980, US 5 043 376, US 5 104 913, US 5 281 659, US 5 194 463, US 4 804 719, WO 92/07913 et EP-A-747036.

A titre illustratif de monomères pour polymères colorant connus, on peut notamment citer les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, les perinones, les nitroarylamines, le benzodifurane, les 2 H-1-benzopyran-2-one, les quinophtalones, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine et oxazine.

L'homme de l'art est bien entendu à même, de par ses connaissances générales, de procéder aux choix des monomères pour ajuster l'effet de couleur recherché selon l'invention.

Le ou les agents de coloration et notamment les pigments mis en oeuvre dans le cadre de la présente invention, peuvent être utilisés soit sous leur forme brute ou sous une forme prétraitée, notamment en leur surface. Ce traitement a généralement pour objectif d'augmenter la stabilité de la couleur et faciliter leur incorporation dans les formulations cosmétiques. En particulier, des agents de coloration, traités en vu de les rendre hydrophobes seront plus facilement dispersibles dans une phase huileuse.

A titre représentatif de ces traitements de surface, on peut notamment citer celui consistant à traiter le pigment avec un agent hydrophobe et oléofuge de type dérivé phosphate perfluoroalkyle comme décrit dans le brevet EP 1 086 683.

De même, il peut être utile de traiter les agents de coloration et notamment les pigments avec un matériau qui les rend compatibles avec les phases huileuses et notamment siliconées utilisées dans les formulations cosmétiques. Des pigments de ce type sont notamment décrits dans le brevet US 5 143 722.

Selon un mode de réalisation particulier, les agents de coloration utilisés selon l'invention sont de couleur jaune, orange, brune ou rouge.

A titre illustratif des agents de coloration convenant plus particulièrement à l'invention, on peut notamment citer les oxydes de fer brun et de fer jaune, enrobés de phosphate de perfluoroalkyle et l'oxyde de titane traité alumine, enrobé de phosphate de perfluoroalkyle, ou l'oxyde de titane enrobé de phosphate de perfluoroalkyle, comme en particulier les pâtes pigmentaires commercialisées, sous les dénominations commerciales YELLOW IRON OXYDE COVAFLUOR, PF5 YELLOW 601 (jaune) et PF5 RED R516L (rouge), PF 5 BLACK BL100 par la société DAITO, sous les dénominations commerciales FA50DRF, FA50DYF, FA65DF et FA65DBF par la société KOBO, le bleu d'outremer enrobé de phosphate de perfluoroalkyle sous la dénomination commerciale PF 5 Ultramarine n° 801 par la société DAITO, les sels disodiques de Tartrazine et les laques d'aluminium du rouge Allura sur alumine commercialisées par la société NOVEON sous les dénominations FDC YELLOW n° 6, A1 Laque et FDC YELLOW N° 5 A1 Laque et leurs mélanges.

Particules réfléchissantes

Par « particules réfléchissantes », on désigne au sens de la présente invention des particules dont la taille, la structure, notamment l'épaisseur de la ou des couches qui la constituent et leur natures physique et chimique, et l'état de surface, leur permettent de réfléchir la lumière incidente. Cette réflexion peut, le cas échéant, posséder une intensité suffisante pour créer à la surface de la composition de type fond de teint selon l'invention, lorsque cette dernière est appliquée sur le support à maquiller, des points de surbrillance visibles à l'oeil nu, c'est-à-dire des points plus lumineux qui contrastent avec leur environnement en semblant briller.

Les particules réfléchissantes sont également sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Les particules réfléchissantes peuvent être présentes dans la composition selon l'invention et la composition de base ou de surface du produit selon l'invention, à une teneur allant de 0,5 % à 60 % par rapport au poids total de la composition, notamment de 1 % à 30 % en poids, en particulier de 2 % à 20 % en poids, voire de 3 % à 10 % en poids.

Ces particules peuvent présenter des formes variées. Ces particules peuvent être notamment en forme de plaquettes ou globulaires, en particulier sphériques.

Les particules réfléchissantes quelque soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, par exemple des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou composé métallique. Le substrat peut être monomatière, multimatériau, organique et/ou inorganique.

Plus particulièrement, il peut être choisi par les verres, les céramiques, le graphite, les oxydes métalliques, les alumines, les silices, les silicates, notamment les aluminosilicates et les borosilicates, le mica synthétique et leurs mélanges, cette liste n'étant pas limitative.

Le matériau réfléchissant peut comporter une couche de métal ou d'un composé métallique.

Des particules de verre recouvertes d'une couche métallique sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent, encore appelées « nacres blanches ».

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelle que soit leur forme, peuvent également être choisies parmi les particules à substrat synthétique enrobé au moins partiellement d'au moins une couche d'au moins un composé métallique, notamment un oxyde métallique, choisi par exemple parmi les oxydes de titane, notamment $TiO_2$, de fer notamment $Fe_2O_3$, d'étain, de chrome, le sulfate de baryum et les composés suivants : $MgF_2$, $CrF_3$, $ZnS$, $ZnSe$, $SiO_2$, $Al_2O_3$, $MgO$, $Y_2O_3$, $SeO_3$, $SiO$, $HfO_2$, $ZrO_2$, $CeO_2$, $Nb_2O_5$, $Ta_2O_5$, $MoS_2$ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD.

Les particules réfléchissantes peuvent être ou non goniochromatiques et/ou interférentielles ou non. Elles comprennent au sens de l'invention, les nacres et les agents de coloration goniochromatiques.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

On peut également envisager d'utiliser à titre de particules réfléchissantes, un agent de coloration goniochromatique sous réserve que cet agent réponde à l'exigence d'effet de teinte requise selon l'invention et ne perturbe pas, par ailleurs, la perception visuelle de la composition en terme d'effet couleur. Cet agent de coloration goniochromatique peut être notamment choisi parmi les structures multicouche interférentielles.

Charges

Comme précisé précédemment, la présence de particules réfléchissantes en proportion suffisante pour assurer une fonction de charge permet de réduire significativement la quantité voire d'éviter la présence de charge(s) conventionnelle(s) de type particules blanches.

En l'occurrence, les compositions de type fond de teint conformes à l'invention peuvent contenir moins de 5 % voire 3 % en poids en particules blanches et notamment d'oxyde de titane, voire peuvent être exemptes en oxyde de titane.

Bien entendu, il demeure toutefois possible d'associer aux particules réfléchissantes selon l'invention une ou plusieurs autres charges conventionnelles sous réserve que celles-ci soient utilisées en une quantité telle qu'elles ne viennent pas affecter l'effet esthétique recherché par les composition de type fond de teint revendiquées c'est-à-dire ne confèrent pas outre mesure un aspect grisâtre à la peau maquillée lorsque celle-ci est revêtue d'une composition conforme à l'invention. L'homme de l'art est à même, de par ses connaissances, de procéder à cet ajustement.

Ces charges peuvent être minérales ou organiques de toute forme, plaquettaires, sphériques ou oblongues, quelque soit la forme cristallographique (par exemple feuillet, cubique, hexagonale, orthorhombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) ou de polyméthacrylate de méthyle (Covabead de Wackherr), les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium et leurs mélanges.

Il peut être avantageux de privilégier le choix de charges complémentaires, transparentes, comme par exemple la silice pyrogénée.

Cette ou ces charges peuvent être présentes à raison de 0,1 à 20 % en poids, de préférence 2 à 15 % en poids, et mieux de 2 % à 10 % en poids par rapport au poids total de la composition de type fond de teint, notamment des compositions de base ou de surface du produit de type fond de teint.

Milieu physiologiquement acceptable

Par « milieu physiologiquement acceptable », on désigne un milieu non toxique et susceptible d'être appliqué sur la peau d'êtres humains. Le milieu physiologiquement acceptable est généralement adapté à la nature de la peau sur laquelle doit être appliquée la composition de type fond de teint ainsi qu'à la forme sous laquelle la composition est destinée à être conditionnée, notamment fluide à la température ambiante et sous pression atmosphérique.

Comme précisé précédemment, les composition de type fond de teint selon l'invention, notamment les compositions de base et/ou de surface du produit de type fond de teint selon l'invention, peuvent être formulées sous une forme fluide ou solide de type poudre, libre, compacte ou coulée.

Elles peuvent notamment, indépendamment l'une de l'autre, se présenter sous une forme anhydre ou sous la forme d'un gel, d'émulsion directe, inverse ou multiple associant au moins une phase aqueuse et au moins une phase grasse.

Phase aqueuse

La composition de type fond de teint selon l'invention, notamment la composition de base et/ou la composition de surface du produit de type fond de teint selon l'invention, peut comprendre au moins un milieu aqueux, constituant une phase aqueuse, qui peut former la phase continue de la composition de type fond de teint considérée.

La phase aqueuse peut être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 %, en poids par rapport au poids total de la composition considérée.

Un tel milieu peut comprendre en outre une huile volatile telle que définie ci-après.

Phase grasse

La composition de type fond de teint selon l'invention, notamment la composition de base et/ou la composition de surface du produit de type fond de teint selon l'invention, peut comporter une phase grasse et notamment au moins un corps gras liquide à température ambiante (25°C) et/ou un corps gras solide à température ambiante tel que les cires, les corps gras pâteux, les gommes et leurs mélanges. La phase grasse peut en outre contenir des solvants organiques lipophiles.

La composition de type fond de teint peut posséder par exemple une phase grasse continue, pouvant contenir moins de 5% d'eau, notamment moins de 1% d'eau par rapport à son poids total et en particulier être sous forme anhydre.

La phase grasse de la composition selon l'invention peut notamment comprendre, à titre de corps gras liquide, au moins une huile volatile ou non volatile ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées, des huiles siliconées, ou leurs mélanges. On entend par "huile hydrocarbonée", une huile contenant principalement des atomes d'hydrogène et de carbone et éventuellement des atomes d'oxygène, d'azote, de soufre, de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls®, les esters ramifiés en $C_8$-$C_{16}$ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10$^{-6}$ m$^2$/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans une composition selon l'invention à une teneur allant de 0,1 à 98 % en poids, notamment de 1 % à 65 % en poids, et en particulier de 2 % à 50 % en poids, par rapport au poids total de la composition.

Les huiles non volatiles peuvent notamment être choisies parmi les huiles hydrocarbonées fluorées et/ou siliconées non volatiles.

Comme huile hydrocarbonée non volatile, on peut notamment citer :

- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810, 812 et 818® par la société Dynamit Nobel,

- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq$ 10, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles non volatiles peuvent être présentes dans une composition selon l'invention en une teneur allant de 0,01 à 90 % en poids, notamment de 0,1 à 85 % en poids, et en particulier de 1 % à 70 % en poids, par rapport au poids total de la composition.

Plus généralement, le corps gras liquide peut être présent à raison de 0,01 à 90 % en poids et notamment de 0,1 à 85 % en poids, par rapport au poids de la phase grasse.

En ce qui concerne le corps gras solide à température ambiante et à pression atmosphérique, il peut être choisi parmi les cires, les corps gras pâteux, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Ainsi, une composition selon l'invention peut comprendre au moins un composé gras pâteux à température ambiante.

Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition de type fond de teint selon l'invention, on peut citer les lanolines et les dérivés de lanoline comme les lanolines acétylées, les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycéride d'origine végétale, on peut utiliser les dérivés d'huile de ricin hydrogénée, tels que le « THIXINR » de Rheox.

On peut aussi citer les corps gras pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société Dow Corning sous les noms commerciaux de DC2503® et DC25514®, et leurs mélanges.

Le corps gras pâteux peut être présent dans une composition selon l'invention en une teneur allant de 0,01

à 50% en poids, de préférence allant de 0,1 à 45 % en poids, et mieux allant de 0,2 % à 30 % en poids, par rapport au poids total de ladite composition.

La composition de type fond de teint selon l'invention, notamment la composition de base et/ou la composition de surface du produit de type fond de teint selon l'invention, peut comprendre en outre une cire. La cire peut être solide à température ambiante (25°C), à changement d'état solide/liquide réversible, ayant une température de fusion supérieure à 30°C pouvant aller jusqu'à 200°C, une dureté supérieure à 0,5MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candellila, les cires de paraffine, l'huile de ricin hydrogénée, les cires de silicone, les cires microcristallines, et leurs mélanges.

En particulier, la cire peut être présente sous forme d'émulsion cire-dans-eau.

La cire peut être présente dans une composition selon l'invention en une teneur allant de 0,01 % à 50 % en poids, en particulier de 0,1 % à 30 % en poids, et notamment de 0,2 % à 20 % en poids, par rapport au poids total de la composition.

Agents tensioactifs

La composition de type fond de teint selon l'invention, notamment la composition de base et/ou la composition de surface du produit de type fond de teint selon l'invention, peut en outre contenir des agents tensioactifs émulsionnants présents notamment en une proportion allant de 0,1 à 30 % en poids, et mieux de 5 % à 15 % en poids, par rapport au poids total de la composition.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition de type fond de teint selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges,
- parmi les tensioactifs anioniques : les acides gras en $C_{16}$-$C_{30}$ neutralisés par les amines, l'ammoniaque ou les sels alcalins, et leurs mélanges.

On utilise de préférence des tensioactifs permettant l'obtention d'émulsion huile-dans-eau ou cire-dans-eau.

Polymère filmogène

La composition de type fond de teint selon l'invention, notamment la composition de base et/ou la composition de surface du produit de type fond de teint selon l'invention, peut comprendre, en outre, au moins un polymère filmogène.

Dans la présente demande, on entend par « polymère filmogène », un polymère apte à former à lui seul ou en présence d'un agent auxiliaire de filmification, un film continu et adhérent sur la peau.

On utilise de préférence un polymère filmogène apte à former un film hydrophobe, c'est-à-dire un polymère dont le film a une solubilité dans l'eau à 25 °C inférieure à 1 % en poids.

Le polymère filmogène peut notamment être au moins un polymère choisi parmi le groupe comprenant :

- les polymères filmogènes hydrosolubles,
- des dispersions aqueuses de particules de polymères filmogènes hydrodispersibles, encore appelées « latex » ; dans ce cas, la composition de type fond de teint doit comprendre une phase aqueuse,
- des polymères filmogènes liposolubles,
- les polymères filmogènes lipodispersibles sous forme de dispersions non aqueuses de particules de polymère, de préférence des dispersions de particules polymériques, le cas échéant stabilisées en leur surface par au moins un agent stabilisant, dans une ou plusieurs huiles de silicones et/ou hydrocarbonées ; ces dispersions non aqueuses sont encore appelées « NAD ».

La composition de type fond de teint peut comprendre parallèlement un mélange de ces polymères.

Le polymère filmogène peut être présent dans une composition selon l'invention en une teneur en matières sèches allant de 0,01 % à 20 % en poids par rapport au poids total de la composition et notamment de 0,5 % à 10 % en poids.

Parmi les polymères filmogènes utilisables selon l'invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en C1-C20, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styréniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Dans un premier exemple de réalisation de la composition de type fond de teint selon l'invention, le polymère filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou ben encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425®, AVALURE UR-450® , SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Dans un second exemple de réalisation de la composition de type fond de teint selon l'invention, le polymère filmogène peut être un polymère hydrosoluble et est donc présent dans la phase aqueuse de la composition sous forme solubilisée. Comme exemples de polymères filmogènes hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels que l'acide hyaluronique, les chondroïtines sulfate, et leurs mélanges.

Selon une autre variante de réalisation de la composition de type fond de teint selon l'invention, le polymère filmogène peut être présent dans une phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées précédemment.

Dans un troisième exemple de réalisation de la composition de type fond de teint selon l'invention, le polymère filmogène peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquencé, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Dans un quatrième exemple de réalisation de la composition de type fond de teint selon l'invention, le polymère filmogène peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui ont pour but qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut en outre comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition ou le produit de type fond de teint de l'invention se présente généralement sous la forme d'un fond de teint notamment à appliquer sur le visage ou le cou, d'un produit anti-cernes, d'un correcteur de teint, d'une crème teintée ou base de maquillage pour le visage ou d'une composition de maquillage pour le corps.

La composition de type fond de teint de l'invention, notamment la composition de base et/ou la composition de surface du produit de type fond de teint selon l'invention, peut se présenter sous une forme solide, par exemple pulvérulente, compactée ou coulée ou sous forme stick ou sous la forme d'un fluide par exemple pâteux ou liquide. Elle peut aussi se présenter sous forme de pâte souple, d'un onguent, d'une pommade solide ou fluide de type crème. Par exemple, elle peut être une émulsion huile-dans-eau ou eau-dans-huile, un gel notamment anhydre, solide ou souple et même sous forme biphasique. Selon cette variante, elle se présente plus particulièrement sous forme d'un fond de teint à phase continue huileuse et notamment anhydre ; dans ce cas, elle peut contenir une phase aqueuse à un taux inférieur à 5 %.

Dans le cas du produit selon l'invention, les deux compositions correspondantes peuvent se présenter sous des formes identiques ou différentes et, notamment conformes à ce qui précède.

La composition de type fond de teint selon l'invention, notamment la composition de base et/ou la composition de surface du produit de type fond de teint selon l'invention, peut être fabriquée par les procédés connus, généralement utilisés dans le domaine cosmétique.

Exemples

Des exemples de formulations de compositions éclaircissantes sont donnés ci-après.
Les proportions sont massiques.

Composition A

- polyméthylcétyldiméthylméthylsiloxane oxyéthyléné (ABIL EM 90 de la société GOLDSCHMIDT)       0,80 %
- iso-stéarate de polyglycérol (4 moles) (ISOLAN GI 34 de la société GOLDSCHMIDT)       0,60 %

- laurate d'hexyle      0,60 %
- polydiméthylsiloxane oxyéthyléné (DP : 70 - Viscosité : 500 CST) (KF-6017 de la société SHIN ETSU SILICONES)      4,48 %
- isoéicosane (PERMETHYL 102 A de PERMETHYL)      2 %
- polydiméthylsiloxane (viscosité : 5 CST) (FLUID 200 5 CS de la société DOW CORNING)      2 %
- néopentanoate d'iso-stéaryle      0,50 %
- cyclohéxadiméthylsiloxane      8 %
- cyclopentadiméthylsiloxane      11,36%
- iso-dodécane      13 %
- D,L-alpha-tocophérol (VITAMINE E)      0,08 %
- hectorite modifiée par du chlorure de distéaryl di-méthyl ammonium (BENTONE 38V de la société ELEMENTIS)      1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO)      1,86 %
- oxyde de fer brun enrobé de phosphate de perfluoroalkyle en dispersion à 50 % en poids dans cyclométhicone/diméthyl polysiloxane copolyol (CI : 77491) (FA50DRF de la société KOBO)      0,72 %
- oxyde de fer noir enrobé de phosphate de perfluoroalkyle en dispersion à 65 % en poids dans cyclométhicone/diméthyl polysiloxane copolyol (CI : 77499) (FA65DBF de la société KOBO)      0,34 %
- oxyde de titane traité alumine enrobé de phosphate de perfluoroalkyle en dispersion à 65 % en poids dans décaméthylcyclopentasiloxane / diméthiconecopolyol (CI 77891) (FA65DF de la société KOBO)      7,39 %
- microsphères creuses de polyméthacrylate de méthyle (granulométrie : 10 à 12 microns), (COVABEAD LH 85 de la société WACKHERR)      4 %
- butylèneglycol-1,3      10 %
- chlorure de sodium      0,70 %
- conservateurs      0,90 %
- mica-oxyde de fer brun (60/40) (CI : 77019 + 77491) (COLORONA PASSION ORANGE de la société MERCK)      2 %
- sel di-sodique de tartrazine (CI : 19140) (FD & C YELLOW 5 de la société LCW)      2 %
- eau      quantité suffisante pour (qsp) 100 %

Composition B

Il s'agit d'un fond de teint convenant plus particulièrement aux peaux foncées claires, sa formulation est la suivante :

- polyméthylcétyldiméthylméthylsiloxane oxyéthyléné (ABIL EM 90 de la société GOLDSCHMIDT)      0,80 %
- iso-stéarate de polyglycérol (4 moles) (ISOLAN GI 34 de la société GOLDSCHMIDT)      0,60 %
- laurate d'hexyle      0,60 %
- polydiméthylsiloxane oxyéthyléné (DP : 70 - Viscosité : 500 CST) (KF-6017 de la société SHIN ETSU SILICONES)      4,48 %
- isoéicosane (PERMETHYL 102A de la société PERMETHYL)      2 %
- polydiméthylsiloxane (viscosité : 5 CST) (FLUID 200 5 CS de la société DOW CORNING)      2 %
- néopentanoate d'iso-stéaryle      0,50 %
- cyclohéxadiméthylsiloxane      8 %
- cyclopentadiméthylsiloxane      11,36%
- iso-dodécane      13 %
- D,L-alpha-tocophérol (VITAMINE E)      0,08 %
- hectorite modifiée par du chlorure de distéaryl di-méthyl ammonium (BENTONE 38V de la société ELEMENTIS)      1,60 %

- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO)      6,90 %
- mica-oxychlorure de bismuth-oxyde de fer brun (47/28/25) (CHROMA - LITE BROWN de la société ENGELHARD)      6,81 %
- laque d'aluminium du rouge Allura sur alumine (40/60) (FD&C Red 40 Al lake de la société NOVEON)      0,60 %
- microsphères creuses de polyméthacrylate de méthyle (granulométrie : 10 à 12 microns), (COVABEAD LH 85 de la société WACKHERR)      4 %
- butylèneglycol-1,3      10 %

- chlorure de sodium        0,70 %
- conservateurs        0,90 %
- eau        qsp 100 %

Composition C

Cette composition convient tout particulièrement aux peaux noires, sa formulation est la suivante :

- polyméthylcétyldiméthylméthylsiloxane oxyéthylène (ABIL EM 90 de la société GOLDSCHMIDT)        0,80 %
- iso-stéarate de polyglycérol (4 moles) (ISOLAN GI 34 de la société GOLDSCHMIDT)        0,60 %
- laurate d'hexyle        0,60 %
- polydiméthylsiloxane oxyéthyléné (DP : 70 - Viscosité : 500 CST) (KF-6017 de la société SHIN ETSU SILICONES)        4,48 %
- isoéicosane (PERMETHYL 102A)        2 %
- polydiméthylsiloxane (viscosité : 5 CST) (FLUID 200 5 CS de la société DOW CORNING)        2 %
- néopentanoate d'iso-stéaryle        0,50 %
- cyclohéxadiméthylsiloxane        8 %
- cyclopentadiméthylsiloxane        11,36%
- iso-dodécane        13 %
- D,L-alpha-tocophérol (VITAMINE E)        0,08 %
- hectorite modifiée par du chlorure de distéaryl di-méthyl ammonium (BENTONE 38V de la société ELEMENTIS)        1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO)        1,86 %
- oxyde de fer brun enrobé de phosphate de perfluoroalkyle en dispersion à 50 % en poids dans cyclométhicone/diméthyl polysiloxane copolyol (CI : 77491) (FA50DRF de la société KOBO)        0,72 %
- oxyde de fer noir enrobé de phosphate de perfluoroalkyle en dispersion à 65 % en poids dans cyclométhicone/diméthyl polysiloxane copolyol (CI : 77499) (FA65DBF de la société KOBO)        0,34 %
- oxyde de titane traité alumine enrobé de phosphate de perfluoroalkyle en dispersion à 65 % en poids dans décaméthylcyclopentasiloxane/diméthiconecopolyol (CI 77891) (FA65DF de la société KOBO)        7,39 %
- microsphères creuses de polyméthacrylate de méthyle (granulométrie : 10 à 12 microns), (COVABEAD LH 85 de la société WACKHERR) g4 %
- butylèneglycol-1,3        10 %
- chlorure de sodium        0,70 %
- conservateurs        0,90 %
- silice-oxyde de fer brun (XIRONA INDIAN SUMMER de MERCK)        4 %
- eau        qsp 100 %

Composition D

Il s'agit d'une composition fond de teint convenant plus particulièrement aux peaux noires, sa formulation est la suivante :

- polyméthylcétyldiméthylméthylsiloxane oxyéthyléné (ABIL EM 90 de la société GOLDSCHMIDT)        0,80 %
- iso-stéarate de polyglycérol (4 moles) (ISOLAN GI 34 de la société GOLDSCHMIDT)        0,60 %
- laurate d'hexyle        0,60 %
- polydiméthylsiloxane oxyéthyléné (DP : 70 - Viscosité : 500 CST) (KF-6017 de la société SHIN ETSU SILICONES)        4,48 %
- isoéicosane (PERMETHYL 102A)        2 %
- polydiméthylsiloxane (viscosité : 5 CST) (FLUID 200 5 CS de la société DOW CORNING)        2 %
- néopentanoate d'iso-stéaryle        0,50 %
- cyclohéxadiméthylsiloxane        8 %
- cyclopentadiméthylsiloxane        11,36%
- iso-dodécane        13 %
- D,L-alpha-tocophérol (VITAMINEE)        0,08 %
- hectorite modifiée par du chlorure de distéaryl di-méthyl ammonium (BENTONE 38V de la société ELEMENTIS)        1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO)     5,25 %
- oxyde de fer brun enrobé de phosphate de perfluoroalkyle en dispersion à 50 % en poids dans cyclométhicone/ diméthyl polysiloxane copolyol (CI : 77491 ) (FA50DRF de la société KOBO)     0,72 %
- oxyde de fer noir enrobé de phosphate de perfluoroalkyle en dispersion à 65 % en poids dans cyclométhicone/ diméthyl polysiloxane copolyol (CI : 77491 ) (FA65DBF de la société KOBO)     0,34 %
- oxyde de titane traité alumine enrobé de phosphate de perfluoroalkyle en dispersion à 65 % en poids dans décaméthylcyclopentasiloxane/diméthiconecopolyol (CI 77891) (FA65DF de la société KOBO)     4 %
- microsphères creuses de polyméthacrylate de méthyle (granulométrie : 10 à 12 microns), (COVABLEAD LH 85 de la société WACKHERR) 4 %
- butylèneglycol-1,3     10 %
- chlorure de sodium     0,70 %
- silice-oxyde de fer brun (XIRONA INDIAN SUMMER de la société MERCK)     4 %
- conservateurs     0,90 %
- eau     qsp 100 %

Composition E

Il s'agit d'un stick pour peau noire, sa formulation est la suivante :

- cire de polyéthylène (PM : 500) (Polywax 500 de BARECO)     4 %
- homopolymère de l'éthylène (Point de fusion : 79,5 °C) (PERFORMALENE 400 de NEW PHASE TECHNOLOGIE)     8 %
- cyclopentadiméthylsiloxane     5 %
- cyclohéxadiméthylsiloxane     20 %
- iso-dodécane     19 %
- phényl triméthylsiloxy trisiloxane (viscosité : 20 CST - PM : 372) (DC556 de DOW CORNING)     19 %
- microsphères creuses de polyméthacrylate de méthyle (granulomètrie : 10 à 12 microns) (COVABLAD LH85 de WACKHERR)     10 %
- mica-oxyde de fer brun (94/6) (CI : 77019 + 77491) (COSMETICA ORANGE de ENGELHARD)     12 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO)     3 %

Composition F

Il s'agit d'un fond de teint fluide pour peaux noires. Sa formulation est comme suit :

- cétyl polyéthylène glycol/PPG - 10/1 diméthicone (ABIL EM 90)     0,80 %
- 4-isostérate de polyglycéryle (ISOLAN GI34 de la société GOLDSCHMIDT)     0,60 %
- laurate d'hexyle     0,60 %
- PEG-10 diméthicone (KF6017)     4,48 %
- isoéicosane (PERMETHYL 102A)     2 %
- diméthicone (DC 200 FLUID)     2 %
- néopentanoate d'isostearyle     0,50 %
- cyclohexasiloxane     8 %
- cyclopentasiloxane     11,36%
- isododécane     13 %
- D, L-alpha-tocophérol (VITAMINE E)     0,08 %
- hectorite modifiée par du chlorure de distéaryl di-méthyl ammonium (BENTONE 38V de la société ELEMENTIS)     1,6 %
- oxydes de fer et cyclopentasiloxane et PEG/PPG-18/18 diméthicone et phosphates de fluoroalcool en $C_9$ à $C_{15}$ (FASODYF de KOBO)     4,73 %
- oxydes de fer et cyclopentasiloxane et PEG/PPG-18/18 diméthicone et phosphates de fluoroalcool en $C_9$ à $C_{15}$ (FA50DRF de KOBO)     2,595 %
- oxydes de fer et cyclopentasiloxane et PEG/PPG-18/18 diméthicone et phosphates de fluoroalcool en $C_9$ à $C_{15}$ (FA65DBF de KOBO)     1,22 %
- dioxyde de titane et cyclopentasiloxane et PEG/PPG-18/18 diméthicone et phosphates de fluoroalcool en $C_9$ à $C_{15}$ et alumine (FA65DF de KOBO) 1,765 %
- polyméthacrylate de méthyl (COVABEAD LH 85)     4 %

- butylène glycol    10 %
- chlorure de sodium    0,70 %
- conservateurs    0,90 %
- mica et oxydes de fer (COLORONA PASSION ORANGE de MERCK)    2 %
- lake yellow 6 (pigments : FDC YELLOW 6 A1 LAKE DE SUN CHEMICAL) (CI15985)    2 %
- eau    qsp 100 %

Composition Y

Il s'agit d'un fond de teint plus particulièrement destiné aux peaux foncées, sa formulation est comme suit :

- polyméthylcétyldiméthylméthylsiloxane oxyéthyléné (ABIL EM 90 de la société GOLDSCHMIDT)    0,80 %
- iso-stéarate de polyglycérol (4 moles) (ISOLAN GI 34 de la société GOLDSCHMIDT)    0,60 %
- laurate d'hexyle    0,60 %
- polydiméthylsiloxane oxyéthyléné (DP : 70 - Viscosité : 500 CST) (KF-6017 de la société SHIN ETSU SILICONES)    4,48 %
- isoeicosane PERMETHYL 102 A de PERMETHYL    2 %
- polydiméthylsiloxane (viscosité : 5 CST) (FLUID 200 5 CST de la société DOW CORNING)    2 %
- néopentanoate d'iso-stéaryle    0,5 %
- cyclohéxadiméthylsiloxane    8 %
- cyclopentadiméthylsiloxane    11,3%
- iso-dodécane    13 %
- D,L-alpha-tocophérol (VITAMINE E)    0,08 %
- hectorite modifiée par du chlorure de distéaryl di-méthyl ammonium (BENTONE 38V de la société ELEMENTIS)    1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO)    10,93 %
- oxyde de fer brun enrobé de phosphate de perfluoroalkyle en dispersion à 50 % en poids dans cyclométhicone/diméthyl polysiloxane copolyol (CI : 77491 ) (FA50DRF de la société KOBO)    2,15 %
- microsphères creuses de polyméthacrylate de méthyle (granulométrie : 10 à 12 microns), (COVABEAD LH 85 de la société WACKHERR)    4 %
- mica-bleu ferrique vendu sous la dénomination MICRONA MATTE BLUE par la société MERCK    1,23 %
- butylèneglycol-1,3    10 %
- chlorure de sodium    0,70 %
- conservateurs    0,90 %
- eau    qsp 100 %

Composition Z

Il s'agit d'un fond de teint pour peaux foncées.

- polyméthylcétyldiméthylméthylsiloxane oxyéthyléné (ABIL EM 90 de la société GOLDSCHMIDT)    0,80 %
- iso-stéarate de polyglycérol (4 moles) (ISOLAN GI 34 de la société GOLDSCHMIDT)    0,60 %
- laurate d'hexyle    0,60 %
- polydiméthylsiloxane oxyéthyléné (DP : 70 - Viscosité : 500 CST) (KF-6017 de la société SHIN ETSU SILICONES)    4,70 %
- isoeicosane PERMETHYL 102 A de PERMETHYL    2 %
- polydiméthylsiloxane (viscosité : 5 CST) (FLUID 200 5 CST de la société DOW CORNING)    2 %
- néopentanoate d'iso-stéaryle    0,50 %
- cyclohéxadiméthylsiloxane    8,4 %
- cyclopentadiméthylsiloxane    11,90 %
- iso-dodécane    13,60 %
- D,L-alpha-tocophérol (VITAMINE E)    0,08 %
- hectorite modifiée par du chlorure de distéaryl di-méthyl ammonium (BENTONE 38V de la société ELEMENTIS)    1,67 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO)    4,73 %
- oxyde de fer brun enrobé de phosphate de perfluoroalkyle en dispersion à 50 % en poids dans cyclométhicone/diméthyl polysiloxane copolyol (CI : 77491) (FA50DRF de la société KOBO)     2,60 %
- oxyde de fer noir enrobé de phosphate de perfluoroalkyle en dispersion à 65 % en poids dans cyclométhicone/ diméthyl polysiloxane copolyol (CI : 77499) (FA65DBF de la société KOBO)     1,20 %
- oxyde de titane traité alumine enrobé de phosphate de perfluoroalkyle en dispersion à 65 % en poids dans décaméthylcyclopentasiloxane/diméthicone copolyol (CI 77891) (FA65DF de la société KOBO)     1,20 %
- microsphères creuses de polyméthacrylate de méthyle (granulométrie : 10 à 12 microns), (COVABEAD LH 85 de la société WACKHERR) 4 %
- butylèneglycol-1,3     10 %
- chlorure de sodium     0,70 %
- conservateurs     0,90 %
- eau     qsp 100 %

Composition G

- polymethylcétyldiméthylméthylsiloxane oxyethyléné (ABIL EM 90 de la société GOLDSCHMIDT)     0,80 %
- iso-stéarate de polyglycérol (4 moles) (ISOLAN GI34 de la société GOLDSCHMIDT)     0,60 %
- laurate d'hexyle     0,60 %
- polydiméthylsiloxane oxyethyléné (DP : 70 - Viscosité : 500 CST) (KF-6017 de la société SHIN ETSU SILICONES)     4,48 %
- isoéicosane (PERMETHYL 102A de PERMETHYL)     2 %
- polydimethylsiloxane (viscosité 5 CST) (FLUID 200 5 CS de la société DOW CORNING)     2 %
- néopentanoate d'iso-stéaryle     0,5 %
- cyclohexadimethylsiloxane     8 %
- isododécane     13 %
- D,L-alpha-tocophérol (VITAMINE E)     0,08 %
- Smectite: silicate de magnésium modifié dans cyclopentadiméthylsiloxane et éthanol (GEL DE BENTONE VS 5 V)     12,96 %
- Oxyde de fer jaune enrobé de phosphate de perfluoroalkyle (PF 5 YELLOW 601 de chez DAITO)     1,28 %
- Oxyde de fer brun enrobé de phosphate de perfluoroalkyle (PF 5 RED R 516 L de chez DAITO)     2,13 %
- Oxyde de fer noir enrobé de phosphate de perfluoroalkyle (PF 5 BLACK BL 100 de chez DAITO)     0,47 %
- Bleu d'outremer enrobé de phosphate de perfluoroalkyle (PF 5 ULTRAMARINE N° 801 de chez DAITO)     1,28 %
- cyclopentadimethylsiloxane (DOW CORNING 245 FLUID de chez DOW CORNING)     5,17 %
- microsphères creuses de polymethacrylate de methyle (granulométrie 10 à 12 μm), (COVABEAD LH85 de la société WACKHERR)     4 %
- butylèneglycol-1,3     10 %
- chlorure de sodium     0,7 %
- conservateurs     0,90 %
- phenoxyethanol (PHENOXETOL)     0,5 %
- mica-oxyde de fer brun (60/40) (CI : 77019+77491 ) (COLORONA PASSION ORANGE de la société MERCK)     2 %
- lake yellow 6 (pigments : FDC YELLOW 6 A1 LAKE DE SUN CHEMICALS) (CI15985)     2 %
- eau     qsp 100 %

Composition H

- polymethylcétyldiméthylméthylsiloxane oxyethyléné (ABIL EM 90 de la société GOLDSCHMIDT)     0,80 %
- iso-stéarate de polyglycérol (4 moles) (ISOLAN GI34 de la société GOLDSCHMIDT)     0,60 %
- laurate d'hexyle     0,60 %
- polydiméthylsiloxane oxyethyléné (DP : 70 - Viscosité : 500 CST) (KF-6017 de la société SHIN ETSU SILICONES)     4,48 %
- isoéicosane (PERMETHYL 102A de PERMETHYL)     2 %
- polydimethylsiloxane (viscosité 5 CST) (FLUID 200 5 CS de la société DOW CORNING)     2 %
- néopentanoate d'iso-stéaryle     0,5 %

- cyclohexadimethylsiloxane        8 %
- isododécane        13 %
- D,L-alpha-tocophérol (VITAMINE E)        0,08 %
- Smectite: silicate de magnésium modifié dans cyclopentadiméthylsiloxane et éthanol (GEL DE BENTONE VS 5 V)        12,96 %
- Oxyde de fer jaune enrobé de phosphate de perfluoroalkyle (PF 5 YELLOW 601 de chez DAITO)        2,37 %
- Oxyde de fer brun enrobé de phosphate de perfluoroalkyle (PF 5 RED R 516 L de chez DAITO)        1,3 %
- Oxyde de fer noir enrobé de phosphate de perfluoroalkyle (PF 5 BLACK BL 100 de chez DAITO)        0,61 %
- Oxyde de titane enrobé de phosphate de perfluoroalkyle (PF 5 ULTRAMARINE N° 801 de chez DAITO)        0,88 %
- cyclopentadimethylsiloxane (DOW CORNING 245 FLUID de chez DOW CORNING)        5,13 %
- microsphères creuses de polymethacrylate de methyle (granulométrie 10 à 12 μm), (COVABEAD LH85 de la société WACKHERR)        4 %
- butylèneglycol-1,3        10 %
- chlorure de sodium        0,7 %
- conservateurs        0,90 %
- phenoxyethanol (PHENOXETOL)        0,5 %
- mica-oxyde de fer brun (60/40) (CI : 77019+77491) (COLORONA PASSION ORANGE de la société MERCK)        2 %
- lake yellow 6 (pigments : FDC YELLOW 6 A1 LAKE DE SUN CHEMICALS) (CI15985)        2 %
- eau qsp        100 %

Composition I

- polymethylcétyldiméthylméthylsiloxane oxyethyléné (ABIL EM 90 de la société GOLDSCHMIDT)        0,80 %
- iso-stéarate de polyglycérol (4 moles) (ISOLAN GI34 de la société GOLDSCHMIDT)        0,60 %
- laurate d'hexyle        0,60 %
- polydiméthylsiloxane oxyethyléné (DP : 70 - Viscosité : 500 CST) (KF-6017 de la société SHIN ETSU SILICONES)        4,48 %
- isoéicosane (PERMETHYL 102A de PERMETHYL)        2 %
- polydimethylsiloxane (viscosité 5 CST) (FLUID 200 5 CS de la société DOW CORNING)        2 %
- néopentanoate d'iso-stéaryle        0,5 %
- cyclohexadimethylsiloxane        8 %
- isododécane        13 %
- D,L-alpha-tocophérol (VITAMINE E)        0,08 %
- Smectite: silicate de magnésium modifié dans cyclopentadimethylsiloxane et éthanol (GEL DE BENTONE VS 5 V)        12,96 %
- Oxyde de fer jaune enrobé de phosphate de perfluoroalkyle (PF 5 YELLOW 601 de chez DAITO)        3,46 %
- cyclopentadimethylsiloxane (DOW CORNING 245 FLUID de chez DOW CORNING)        3,46 %
- microsphères creuses de polymethacrylate de methyle (granulométrie 10 à 12 μm), (COVABEAD LH85 de la société WACKHERR)        4 %
- butylèneglycol-1,3        10 %
- chlorure de sodium        0,7 %
- conservateurs        0,90 %
- phenoxyethanol (PHENOXETOL)        0,5 %
- Mica-oxychlorure de bismuth-oxyde de fer brun (CHROMA-LITE BROWN 4509 de chez ENGELHARD)        6,81 %
- Laque d'aluminium du rouge allura sur alumine (FDC RED 40 AL LAKE - 6808 de chez NOVEON)        0,6 %
- eau        qsp 100%

Composition J

- polymethylcétyldiméthylméthylsiloxane oxyethyléné (ABIL EM 90 de la société GOLDSCHMIDT)        0,80 %
- iso-stéarate de polyglycérol (4 moles) (ISOLAN GI34 de la société GOLDSCHMIDT)        0,60 %

- laurate d'hexyle        0,60 %
- polydiméthylsiloxane oxyethyléné (DP : 70 - Viscosité : 500 CST) (KF-6017 de la société SHIN ETSU SILICONES)        4,48 %
- isoéicosane (PERMETHYL 102A de PERMETHYL)        2 %
- polydimethylsiloxane (viscosité 5 CST) (FLUID 200 5 CS de la société DOW CORNING)        2 %
- néopentanoate d'iso-stéaryle        0,5 %
- cyclohexadimethylsiloxane        8 %
- isododécane        13 %
- D,L-alpha-tocophérol (VITAMINE E)        0,08 %
- Smectite: silicate de magnésium modifié dans cyclopentadiméthylsiloxane et éthanol (GEL DE BENTONE VS 5 V)        12,96 %
- Oxyde de fer jaune enrobé de phosphate de perfluoroalkyle (PF 5 YELLOW 601 de chez DAITO)        1,75 %
- Oxyde de fer brun enrobé de phosphate de perfluoroalkyle (PF 5 RED R 516 L de chez DAITO)        0,39 %
- Oxyde de fer noir enrobé de phosphate de perfluoroalkyle (PF 5 BLACK BL 100 de chez DAITO)        0,14 %
- Oxyde de titane enrobé de phosphate de perfluoroalkyle (PF 5 TIO2 A 100 de chez DAITO)        2,22 %
- cyclopentadimethylsiloxane (DOW CORNING 245 FLUID de chez DOW CORNING)        4,49 %
- microsphères creuses de polymethacrylate de methyle (granulométrie 10 à 12 µm), (COVABEAD LH85 de la société WACKHERR)        4 %
- butylèneglycol-1,3        10 %
- chlorure de sodium        0,7 %
- conservateurs        0,90 %
- phenoxyethanol (PHENOXETOL)        0,5 %
- Silice-oxyde de fer brun (XIRONA INDIAN SUMMER de chez MERCK)        3 %
- Mica-oxychlorure de bismuth-oxyde de fer jaune (CHROMA-LITE YELLOW 4502 de chez ENGELHARD)        2,32 %
- eau        qsp 100 %

Composition K

- polymethylcétyldiméthylméthylsiloxane oxyethyléné (ABIL EM 90 de la société GOLDSCHMIDT)        0,80 %
- iso-stéarate de polyglycérol (4 moles) (ISOLAN GI34 de la société GOLDSCHMIDT)        0,60 %
- laurate d'hexyle        0,60 %
- polydiméthylsiloxane oxyethyléné (DP : 70 - Viscosité : 500 CST) (KF-6017 de la société SHIN ETSU SILICONES)        4,48 %
- isoéicosane (PERMETHYL 102A de PERMETHYL)        2 %
- polydimethylsiloxane (viscosité 5 CST) (FLUID 200 5 CS de la société DOW CORNING)        2 %
- néopentanoate d'iso-stéaryle        0,5 %
- cyclohexadimethylsiloxane        8 %
- isododécane        13 %
- D,L-alpha-tocophérol (VITAMINE E)        0,08 %
- Smectite: silicate de magnésium modifié dans cyclopentadiméthylsiloxane et éthanol (GEL DE BENTONE VS 5 V)        12,96 %
- Oxyde de fer jaune enrobé de phosphate de perfluoroalkyle (PF 5 YELLOW 601 de chez DAITO)        1,75 %
- Oxyde de fer brun enrobé de phosphate de perfluoroalkyle (PF 5 RED R 516 L de chez DAITO)        0,39 %
- Oxyde de fer noir enrobé de phosphate de perfluoroalkyle (PF 5 BLACK BL 100 de chez DAITO)        0,19 %
- Oxyde de titane enrobé de phosphate de perfluoroalkyle (PF 5 TIO2 A 100 de chez DAITO)        2,17 %
- cyclopentadimethylsiloxane (DOW CORNING 245 FLUID de chez DOW CORNING)        4,49 %
- microsphères creuses de polymethacrylate de methyle (granulométrie 10 à 12 µm), (COVABEAD LH85 de la société WACKHERR)        4 %
- butylèneglycol-1,3        10 %
- chlorure de sodium        0,7 %
- conservateurs        0,90 %
- phenoxyethanol (PHENOXETOL)        0,5 %
- Silice-oxyde de fer brun (XIRONA INDIAN SUMMER de chez MERCK)        3,5 %
- Mica-oxychlorure de bismuth-oxyde de fer jaune (CHROMA-LITE YELLOW 4502 de chez ENGELHARD)        1,83 %

-    eau        qsp 100 %

## Mesure de la couleur des compositions

La couleur de chaque composition A, B, C, Y, Z, G, H, I, J, K est donnée dans le tableau suivant, les valeurs étant indiquées avec une précision de 15 %, mieux 10 %, mieux encore 5 % :

| Composition | L* | C* | h | Intervalle |
|---|---|---|---|---|
| A | 39,46 | 41,52 | 63,34° | 30 < L* < 40 |
| B | 48,59 | 37,77 | 57,65° | 40 < L* < 50 |
| C | 57,24 | 24,53 | 53,22° | 50 < L* < 60 |
| Y | 39,22 | 35,08 | 66,83° | 30 < L* < 40 |
| Z | 38,21 | 31,39 | 55,34° | 30 < L* < 40 |
| G | 38,04 | 23,06 | 42,08° | 30 < L* < 40 |
| H | 43,50 | 29,31 | 46,30° | 40 < L* < 50 |
| I | 48,38 | 30,06 | 51,46° | 40 < L* < 50 |
| J | 55,89 | 29,17 | 53° | 50 < L* < 60 |
| K | 55,78 | 27,32 | 51,93° | 50 < L* < 60 |

Pour mesurer la couleur d'une composition, on a procédé à une mesure de couleur dans la masse du produit, comme suit.

On a rempli une cuve référencée H247 avec la composition.

On a arasé puis appliqué sur la composition une lame de verre en prenant soin d'éviter les bulles d'air sous la lame. On a effectué une mesure de couleur avec un spectrocolorimètre de marque MINOLTA® de type CM-2002 en moyenne ouverture et en mode réflexion spéculaire inclus.

Les compositions A, Y, Z et G ont une clarté comprise entre 30 et 40, et sont plus particulièrement adaptées à des peaux foncées de carnation sombre.

Les compositions B, H et I ont une clarté comprise entre 40 et 50, et sont plus particulièrement adaptées à des peaux foncées de carnation moyenne.

Enfin, les compositions C, J et K ont une clarté comprise entre 50 et 60, et elles sont adaptées à des peaux foncées de carnation claire.

On a également effectué une mesure de réflectance spectrale, dans la masse, à l'aide d'un spectrocolorimètre de marque MINOLTA® de type CM3700d, en mode réflexion, spéculaire exclu, UV inclus, et avec une petite ouverture d/8.

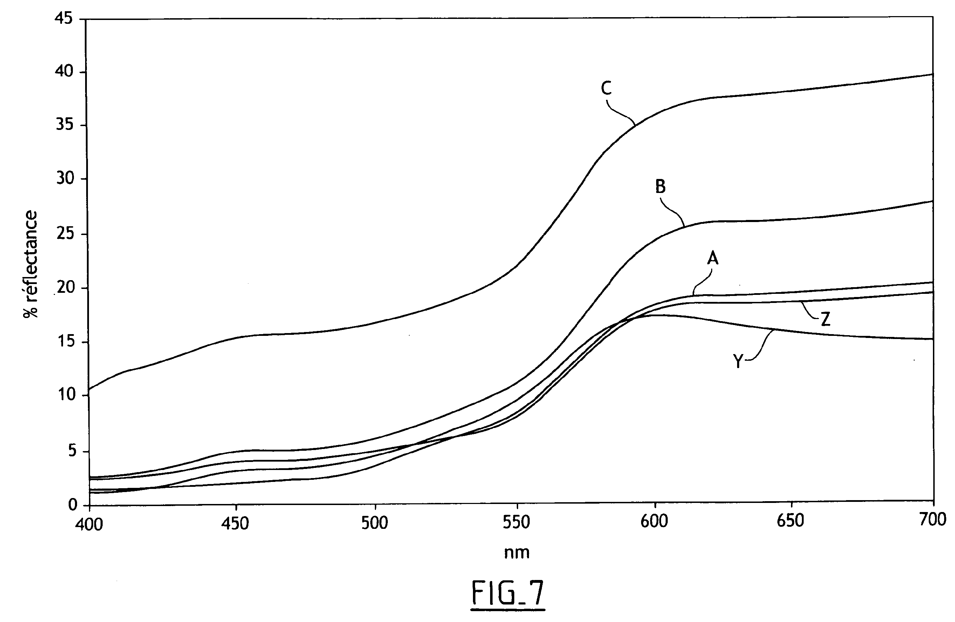

La figure 7 représente les spectres de réflectance des compositions A, B, C, Y et Z présentes dans des coupelles métalliques de quelques centimètres de côté, l'épaisseur de la composition étant de l'ordre d'un centimètre.

On peut remarquer que pour la région de longueurs d'onde comprise entre 600 et 680 nm, la réflectance est comprise entre 10 et 45 %, plus précisément entre 12 et 40 %, pour les cinq compositions testées. La réflectance est inférieure à 20 % dans l'intervalle allant de 450 à 500 nm.

## Essais *in vivo*

Des mesures peuvent être effectuées avant et après maquillage, par exemple au moyen du dispositif d'acquisition décrit en référence à la figure 1, comme décrit plus haut.

On a maquillé avec la composition A des peaux foncées ayant une carnation définie par une clarté L* comprise entre 35 et 42, une saturation C* comprise entre 13 et 20, une position a* sur l'axe rouge/vert comprise entre 9 et 15, une position b* sur l'axe bleu/jaune comprise entre 13 et 17 et une valeur d'angle de teinte h comprise entre 39° et 52°.

On a pu mesurer pour un sujet ayant une clarté de 35,5 et une saturation de 15,3 des clarté et saturation après maquillage respectivement de 37,6 et 16,5, soit des variations de clarté ΔL* de 2,1 et de saturation ΔC* de 1,2. D'autres essais ont montré que la composition A semblait tout à fait adaptée à éclaircir des peaux dont la clarté L* était inférieure à 42 et la saturation C* inférieure à 21.

La composition B a été appliquée sur un sujet ayant une clarté de 44,4 et une saturation de 19,8. On a mesuré des écarts ΔL* et ΔC* respectivement de 0,8 et 0,6 et obtenu un résultat satisfaisant.

La composition C convient davantage aux peaux foncées claires, notamment celles ayant une clarté comprise entre 48 et 54 et une saturation comprise entre 24 et 28.

On a également pu obtenir un maquillage éclaircissant avec les compositions Y et Z.

## Essais sur cartes de contraste

On peut, pour déterminer le pouvoir d'homogénéisation $1/\Delta E_1$ et le pouvoir couvrant $1/\Delta E_2$ d'une composition, utiliser les cartes de contraste décrites plus haut.

En utilisant les cartes de contraste 30, 31 et 32, on a pu caractériser les différentes compositions A, B, C, Y, Z, G, I, J en procédant de la manière suivante.

### Compositions liquides ou pâteuses

On dépose une couche de fond de teint d'une épaisseur de 30 µm avec un étaleur automatique de type BRAIVE INSTRUMENTS, sur un film FOLEX (COPIER FILM) (de référence 117230).

Le film recouvert de fond de teint est séché dans une étuve à 37° pendant 24 heures, puis est superposé à l'une des cartes de contraste 30, 31 ou 32 suivant la clarté du fond de teint.

De préférence, on recouvre la carte de contraste 32 d'une composition adaptée aux carnations foncées sombres, la carte de contraste 31 d'une composition adaptée aux carnations moyennes, et la carte 30 d'une composition adaptée aux carnations claires.

Ainsi, on a pu caractériser les compositions A, Y, Z et G en utilisant la carte de contraste 32.

On a pu caractériser par ailleurs les compositions B, H et I en utilisant la carte de contraste 31.

Enfin, on a pu caractériser les compositions C, J et K en utilisant la carte de contraste 30.

Pour les compositions adaptées aux carnations foncées sombres, on mesure à travers le film recouvert de composition et superposé à la carte de contraste 32 l'écart colorimétrique $\Delta E_{1\,zone/B11+}$ entre d'une part chaque zone B11, B12, B12+, XXX et d'autre part la zone B11+, puis on calcule l'écart moyen $\Delta E_{1\,moyen}$ :

$$\Delta E_{1\,moyen} = (\Delta E_{1\,B11/B11+} + \Delta E_{1\,B12/B11+} + \Delta E_{1\,B12+/B11+} + \Delta E_{1\,XXX/B11+})/4$$

avec

$$\Delta E_{1B11/B11+} = [(a^*_{B11} - a^*_{B11+})^2 + (b^*_{B11} - b^*_{B11+})^2 + (L^*_{B11} - L^*_{B11+})^2]^{1/2}$$

D'une manière générale, on utilisera la notation :

$$\Delta E_{1\,M/N} = [(a_M^* - a_N^*)^2 + (b_M^* - b_N^*)^2 + (L_M^* - L_N^*)^2]^{1/2}$$

On définit le pouvoir d'homogénéisation par $1/\Delta E_{1\,moyen}$.

On mesure également l'écart colorimétrique $\Delta E_2$ entre la zone B11+ et la bordure blanche 34 et l'on définit le pouvoir couvrant par $1/\Delta E_2$.

On a obtenu le résultat suivant avec les compositions A, Y, Z et G :

| Composition | A | Y | Z | G |
|---|---|---|---|---|
| $\Delta E_1$ moyen | 0,24 | 0,36 | 0,62 | 0,68 |
| $\Delta E_2$ | 1,58 | 1,67 | 1,84 | 3,98 |

On peut notamment chercher, en utilisant la carte de contraste, à sélectionner les compositions de manière à avoir un fort pouvoir d'homogénéisation sans que le pouvoir couvrant ne soit trop élevé. Un fort pouvoir d'homogénéisation peut permettre de masquer des défauts de la peau. Un pouvoir couvrant qui n'est pas trop grand peut permettre à la peau de conserver un aspect naturel.

Pour les compositions adaptées aux carnations moyennes, on superpose le film à la carte de contraste 31.

On mesure à travers le film ainsi recouvert de composition l'écart colorimétrique $\Delta E_{1\,zone/C9}$ entre d'une part chaque zone C8, C10, C11 et B12 et d'autre part la zone C9, puis on calcule l'écart moyen $\Delta E_{1\,moyen}$ :

$$\Delta E_{1\ moyen} = (\Delta E_{1\ C8/C9} + \Delta E_{1\ C10/C9} + \Delta E_{1\ C11/C9} + \Delta E_{1\ B12/C9})/4.$$

On mesure également l'écart colorimétrique $\Delta E_2$ entre la zone C9 et la bordure blanche 34 et l'on définit le pouvoir couvrant $1/\Delta E_2$.

On a obtenu les résultats suivants avec les compositions B et I :

| Composition | B | I |
|---|---|---|
| $\Delta E_{1\ moyen}$ | 0,89 | 0,73 |
| $\Delta E_2$ | 8,29 | 6,68 |

Pour les compositions adaptées aux carnations foncées claires, on utilise la carte de contraste 30 et on mesure l'écart colorimétrique $\Delta E_{1\ zone/D6}$ entre d'une part chaque zone D5, D7, D8 et C11 et d'autre part la zone D6, puis on calcule l'écart moyen $\Delta E_{1\ moyen}$ :

$$\Delta E_{1\ moyen} = (\Delta E_{1\ D5/D6} + \Delta E_{1\ D7/D6} + \Delta E_{1\ D8/D6} + \Delta E_{1\ C11/D6})/4.$$

On mesure également l'écart colorimétrique $\Delta E_2$ entre la zone D6 et la bordure blanche 34 et l'on définit le pouvoir couvrant $1/\Delta E_2$.

On a obtenu les résultats suivants avec les compositions C et J :

| Composition | C | J |
|---|---|---|
| $\Delta E_{1\ moyen}$ | 0,77 | 1,07 |
| $\Delta E_2$ | 2,96 | 6,68 |

Par ailleurs, en utilisant la carte représentée sur la figure 5, on a pu caractériser les différentes compositions A, B, C, Y et Z de la manière suivante.

On dépose le fond de teint sur un film transparent avec une épaisseur de $20\mu m$, on laisse sécher 10 min à 37 °C dans une étuve, et l'on superpose ce film à la carte de contraste. On mesure à travers le film ainsi recouvert de composition l'écart colorimétrique $\Delta E_{1\ zone\ Zi/Z6}$ entre d'une part chaque zone Z1, Z2, Z3, Z4 et Z5 et d'autre part la zone Z6, puis on calcule l'écart moyen $\Delta E_{1\ moyen}$ :

$$\Delta E_{1\ moyen} = (\Delta E_{1\ zone\ Z1/Z6} + \Delta E_{1\ zone\ Z2/Z6} + \Delta E_{1\ zone\ Z3/Z6} + \Delta E_{1\ zone\ Z4/Z6} + \Delta E_{1\ zone\ Z5/Z6})/5$$

avec

$$\Delta E_{1\ zone\ Zi/Z6} = [(a^*_{Zi} - a^*_{Z6})^2 + (b^*_{Zi} - b^*_{Z6})^2 + (L^*_{Zi} - L^*_{Z6})^2]^{1/2},$$

pour i = 1, 2, ..., 5.

On définit le pouvoir d'homogénéisation par $1/\Delta E_{1moyen}$.

On mesure également l'écart colorimétrique $\Delta E_2$ entre la zone Z6 de référence et la bordure blanche Z7 et l'on définit le pouvoir couvrant par $1/\Delta E_2$.

On a obtenu les résultats suivants avec les compositions A, B, C, Y et Z :

| Composition | A | B | C | Y | Z |
|---|---|---|---|---|---|
| $\Delta E_{1\ moyen}$ | 1,85 | 2,80 | 1,97 | 2,00 | 1,88 |
| $\Delta E_2$ | 13,56 | 18,73 | 11,11 | 14,98 | 13,92 |

### Autres formes galéniques

Poudres

Dans le cas de poudres compactées, on procède à leur décompactage de manière à les transformer en poudres libres.

On dépose la poudre de manière homogène sur une surface plane puis on applique sur la poudre un film plastique transparent adhésif avec une pression de 100 g/cm$^2$, de manière à ce que la poudre colle à l'adhésif et à obtenir une surface adhésive saturée de poudre. La face adhésive chargée de poudre est ensuite posée contre une plaque de verre transparente et l'ensemble est déposé sur la carte de contraste. Les mesures de couleur des différentes zones de la carte de contraste sont effectuées comme précédemment, au travers du film transparent chargé de composition.

Stick

Le stick peut être fondu de manière à être déposé sous la forme d'une couche de 20 μm d'épaisseur sur un film transparent. On laisse sécher 10 min à 37 °C dans une étuve puis on applique le film sur la carte de contraste, à l'instar de ce qui a été décrit plus haut.

Spray

On dépose une épaisseur de 20 μm de composition et on laisse sécher 10 min à 37 °C dans une étuve.

Témoin coloré sur conditionnement

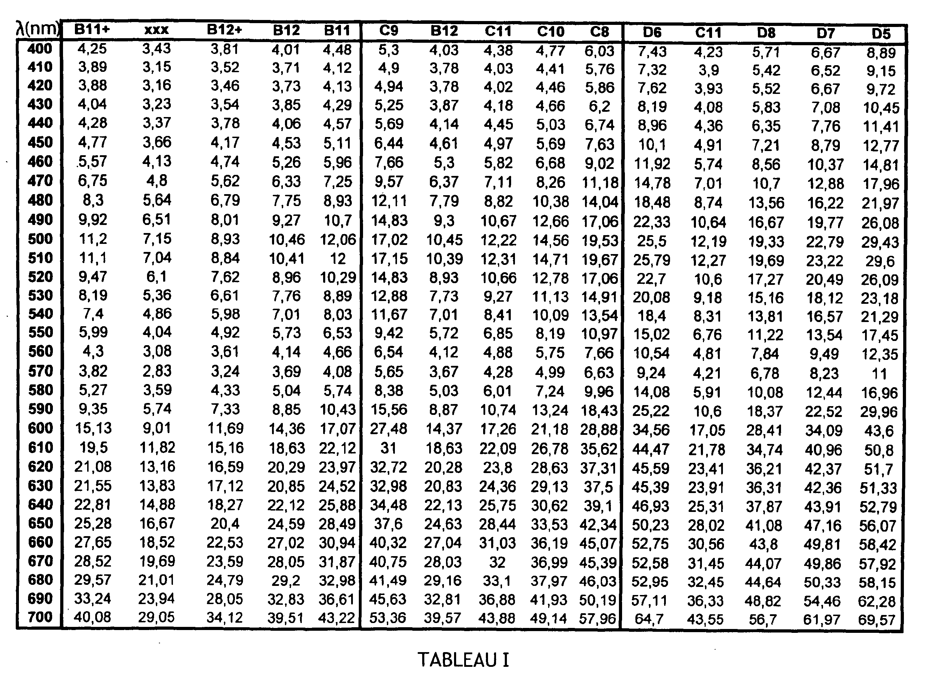

On peut avantageusement faire figurer sur un conditionnement, tel qu'illustré sur la figure 8, contenant la composition, par exemple un étui en carton contenant un récipient rempli avec la composition ou le récipient lui-même, un témoin coloré 20 destiné à permettre au consommateur de déterminer sur le lieu de vente si la composition est adaptée à sa carnation.

Le témoin coloré 20 peut par exemple comporter une ou plusieurs zones colorées 21, 22 et 23 imitant une ou plusieurs couleurs moyennes de peaux pour lesquelles la composition est particulièrement adaptée, par exemple les peaux foncés claires, foncées moyennes ou foncées sombres.

L'une au moins des zones 21, 22 et 23 peut par exemple présenter une clarté L* comprise entre 30 et 60, notamment entre 30 et 55, une saturation C* comprise entre 8 et 30, notamment entre 10 et 30, notamment entre 12 et 28, et une valeur d'angle de teinte h comprise entre 38° et 63°, notamment entre 38° et 54°.

On peut également prévoir sur le lieu de vente un système comportant un capteur, ce système étant agencé d'une part pour mesurer au moins la clarté de la peau, et de préférence également sa saturation, et d'autre part pour délivrer un conseil relatif au choix de la composition permettant d'éclaircir la peau. Le système peut notamment être agencé pour préconiser au moins une composition permettant d'obtenir des variations de clarté ΔL* et de saturation ΔC*, compte tenu des clarté L* et saturation C* de la peau à maquiller, telles que l'on ait

$0,5 \leq \Delta L^* \leq 4$, de préférence $0,5 \leq \Delta L^* \leq 2,5$ et
$0,5 \leq \Delta C^* \leq 4$, de préférence $0,5 \leq \Delta C^* \leq 2,5$.

Dans toute la description, y compris les revendications, l'expression « comportant un » doit être comprise comme étant synonyme de « comportant au moins un », sauf si le contraire est spécifié.

L'expression « compris entre » doit se comprendre bornes incluses, sauf si le contraire est spécifié.

### Revendications

1. Composition de type fond de teint, comportant dans un milieu physiologiquement acceptable, au moins un agent de coloration, **caractérisée par le fait qu'**elle est apte, lorsqu'elle est appliquée sur une peau foncée, à produire une variation positive de clarté ΔL* comprise entre 0,5 et 4 et une variation positive de saturation ΔC* comprise entre 0,5 et 4.

2. Composition selon la revendication 1, **caractérisé par le fait que** ΔL* est compris entre 0,5 et 3 et ΔC* entre 0,5 et 3.

3. Composition selon la revendication 1, **caractérisé par le fait que** ΔL* est compris entre 0,5 et 2,5 et ΔC* entre 0,5 et 2,5.

**4.** Composition selon l'une quelconque des revendications précédentes, **caractérisée par le fait qu'**elle est en outre apte à produire une variation négative d'indice de coxellographie $\Delta i_{cox}$ inférieure ou égale à -0,4, de préférence inférieure ou égale à -0,5, et de préférence encore inférieure ou égale à -1, pour une personne présentant, avant le maquillage, un indice de coxellographie $i_{cox}$ supérieur ou égal à 5.

**5.** Composition selon l'une quelconque des revendications précédentes, **caractérisée par le fait qu'**elle présente une clarté L* supérieure ou égale à 34, une position b* sur l'axe bleu/jaune comprise entre 28 et 42 et une saturation C* comprise entre 31 et 52, mieux une clarté L* comprise entre 34 et 41, une valeur b* comprise entre 33 et 42 et une saturation C* comprise entre 37 et 46.

**6.** Composition selon l'une quelconque des revendications 1 à 4, **caractérisée par le fait qu'**elle présente une clarté L* supérieure ou égale à 34, une position b* selon l'axe bleu/jaune comprise entre 23 et 39 et une saturation C* comprise entre 27 et 47, mieux une clarté L* comprise entre 34 et 48, une position b* comprise entre 27 et 35 et une saturation C* comprise entre 31 et 41.

**7.** Composition selon l'une quelconque des revendications 1 à 4, **caractérisée par le fait qu'**elle présente une clarté L* supérieure ou égale à 34, une position b* sur l'axe bleu/jaune comprise entre 19 et 33 et une saturation C* comprise entre 23 et 40, notamment une clarté L* comprise entre 34 et 42, une position b* comprise entre 23 et 29 et une saturation C* comprise entre 28 et 35.

**8.** Composition selon l'une quelconque des revendication 1 à 4, **caractérisée par le fait qu'**elle présente une clarté L* supérieure ou égale à 39, une position b* sur l'axe bleu/jaune comprise entre 23 et 39 et une saturation C* comprise entre 38 et 41, mieux une clarté L* comprise entre 39 et 48, une position b* comprise entre 28 et 35 et une saturation C* comprise entre 33 et 41.

**9.** Composition selon l'une quelconque des revendication 1 à 4, **caractérisée par le fait qu'**elle présente une clarté L* supérieure ou égale à 51, une position b* sur l'axe bleu/jaune comprise entre 14 et 25 et une saturation C* comprise entre 18 et 31, mieux une clarté L* comprise entre 51 et 63, une position b* comprise entre 14 et 25 et une saturation C* comprise entre 22 et 28.

**10.** Composition selon l'une quelconque des revendications 1 à 4, **caractérisée par le fait qu'**elle présente une clarté L* supérieure ou égale à 34, une position b* selon l'axe bleu/jaune comprise entre 11 et 20 et une saturation C* comprise entre 17 et 29, mieux une clarté L* comprise entre 34 et 48, une position b* comprise entre 14 et 17 et une saturation C* comprise entre 20 et 26.

**11.** Composition selon l'une quelconque des revendications 1 à 10, **caractérisée par le fait qu'**elle constitue un fond de teint liquide.

**12.** Composition selon l'une quelconque des revendications précédentes, **caractérisée par le fait qu'**elle est conditionnée avec un témoin coloré (20) imitant au moins une couleur de peau que la composition est adaptée à éclaircir.

**13.** Utilisation d'une composition telle que définie dans l'une quelconque des revendications 1 à 12 pour éclaircir une peau foncée.

**14.** Utilisation de la composition telle que définie dans la revendication 5 pour éclaircir une peau ayant une carnation définie par une clarté comprise entre 35 et 42 et une saturation comprise entre 13 et 20.

**15.** Utilisation de la composition telle que définie à la revendication 5 pour éclaircir une peau ayant une carnation définie par une clarté inférieure à 42 et une saturation inférieure à 21.

**16.** Utilisation de la composition telle que définie à la revendication 9 pour éclaircir une peau ayant une carnation définie par une clarté comprise entre 48 et 54 et une saturation comprise entre 24 et 28.

**17.** Procédé pour éclaircir une peau foncée, comportant l'application sur la peau d'une composition telle que définie dans l'une quelconque des revendications 1 à 12.

**18.** Procédé de maquillage d'une peau foncée, comprenant l'application sur la peau d'une composition telle que définie dans l'une quelconque des revendications 1 à 12.

FIG.1

FIG.2

FIG.3

FIG.4

FIG.8

10

Z6

Z7

## FIG.5

10'

Z7

Z8

Z6

Z1    Z2    Z3    Z4    Z5

## FIG.6

FIG.7

| λ(nm) | B11+ | xxx | B12+ | B12 | B11 | C9 | B12 | C11 | C10 | C8 | D6 | C11 | D8 | D7 | D5 |
|---|---|---|---|---|---|---|---|---|---|---|---|---|---|---|---|
| 400 | 4,25 | 3,43 | 3,81 | 4,01 | 4,48 | 5,3 | 4,03 | 4,38 | 4,77 | 6,03 | 7,43 | 4,23 | 5,71 | 6,67 | 8,89 |
| 410 | 3,89 | 3,15 | 3,52 | 3,71 | 4,12 | 4,9 | 3,78 | 4,03 | 4,41 | 5,76 | 7,32 | 3,9 | 5,42 | 6,52 | 9,15 |
| 420 | 3,88 | 3,16 | 3,46 | 3,73 | 4,13 | 4,94 | 3,78 | 4,02 | 4,46 | 5,86 | 7,62 | 3,93 | 5,52 | 6,67 | 9,72 |
| 430 | 4,04 | 3,23 | 3,54 | 3,85 | 4,29 | 5,25 | 3,87 | 4,18 | 4,66 | 6,2 | 8,19 | 4,08 | 5,83 | 7,08 | 10,45 |
| 440 | 4,28 | 3,37 | 3,78 | 4,06 | 4,57 | 5,69 | 4,14 | 4,45 | 5,03 | 6,74 | 8,96 | 4,36 | 6,35 | 7,76 | 11,41 |
| 450 | 4,77 | 3,66 | 4,17 | 4,53 | 5,11 | 6,44 | 4,61 | 4,97 | 5,69 | 7,63 | 10,1 | 4,91 | 7,21 | 8,79 | 12,77 |
| 460 | 5,57 | 4,13 | 4,74 | 5,26 | 5,96 | 7,66 | 5,3 | 5,82 | 6,68 | 9,02 | 11,92 | 5,74 | 8,56 | 10,37 | 14,81 |
| 470 | 6,75 | 4,8 | 5,62 | 6,33 | 7,25 | 9,57 | 6,37 | 7,11 | 8,26 | 11,18 | 14,78 | 7,01 | 10,7 | 12,88 | 17,96 |
| 480 | 8,3 | 5,64 | 6,79 | 7,75 | 8,93 | 12,11 | 7,79 | 8,82 | 10,38 | 14,04 | 18,48 | 8,74 | 13,56 | 16,22 | 21,97 |
| 490 | 9,92 | 6,51 | 8,01 | 9,27 | 10,7 | 14,83 | 9,3 | 10,67 | 12,66 | 17,06 | 22,33 | 10,64 | 16,67 | 19,77 | 26,08 |
| 500 | 11,2 | 7,15 | 8,93 | 10,46 | 12,06 | 17,02 | 10,45 | 12,22 | 14,56 | 19,53 | 25,5 | 12,19 | 19,33 | 22,79 | 29,43 |
| 510 | 11,1 | 7,04 | 8,84 | 10,41 | 12 | 17,15 | 10,39 | 12,31 | 14,71 | 19,67 | 25,79 | 12,27 | 19,69 | 23,22 | 29,6 |
| 520 | 9,47 | 6,1 | 7,62 | 8,96 | 10,29 | 14,83 | 8,93 | 10,66 | 12,78 | 17,06 | 22,7 | 10,6 | 17,27 | 20,49 | 26,09 |
| 530 | 8,19 | 5,36 | 6,61 | 7,76 | 8,89 | 12,88 | 7,73 | 9,27 | 11,13 | 14,91 | 20,08 | 9,18 | 15,16 | 18,12 | 23,18 |
| 540 | 7,4 | 4,86 | 5,98 | 7,01 | 8,03 | 11,67 | 7,01 | 8,41 | 10,09 | 13,54 | 18,4 | 8,31 | 13,81 | 16,57 | 21,29 |
| 550 | 5,99 | 4,04 | 4,92 | 5,73 | 6,53 | 9,42 | 5,72 | 6,85 | 8,19 | 10,97 | 15,02 | 6,76 | 11,22 | 13,54 | 17,45 |
| 560 | 4,3 | 3,08 | 3,61 | 4,14 | 4,66 | 6,54 | 4,12 | 4,88 | 5,75 | 7,66 | 10,54 | 4,81 | 7,84 | 9,49 | 12,35 |
| 570 | 3,82 | 2,83 | 3,24 | 3,69 | 4,08 | 5,65 | 3,67 | 4,28 | 4,99 | 6,63 | 9,24 | 4,21 | 6,78 | 8,23 | 11 |
| 580 | 5,27 | 3,59 | 4,33 | 5,04 | 5,74 | 8,38 | 5,03 | 6,01 | 7,24 | 9,96 | 14,08 | 5,91 | 10,08 | 12,44 | 16,96 |
| 590 | 9,35 | 5,74 | 7,33 | 8,85 | 10,43 | 15,56 | 8,87 | 10,74 | 13,24 | 18,43 | 25,22 | 10,6 | 18,37 | 22,52 | 29,96 |
| 600 | 15,13 | 9,01 | 11,69 | 14,36 | 17,07 | 27,48 | 14,37 | 17,26 | 21,18 | 28,88 | 34,56 | 17,05 | 28,41 | 34,09 | 43,6 |
| 610 | 19,5 | 11,82 | 15,16 | 18,63 | 22,12 | 31 | 18,63 | 22,09 | 26,78 | 35,62 | 44,47 | 21,78 | 34,74 | 40,96 | 50,8 |
| 620 | 21,08 | 13,16 | 16,59 | 20,29 | 23,97 | 32,72 | 20,28 | 23,8 | 28,63 | 37,31 | 45,59 | 23,41 | 36,21 | 42,37 | 51,7 |
| 630 | 21,55 | 13,83 | 17,12 | 20,85 | 24,52 | 32,98 | 20,83 | 24,36 | 29,13 | 37,5 | 45,39 | 23,91 | 36,31 | 42,36 | 51,33 |
| 640 | 22,81 | 14,88 | 18,27 | 22,12 | 25,88 | 34,48 | 22,13 | 25,75 | 30,62 | 39,1 | 46,93 | 25,31 | 37,87 | 43,91 | 52,79 |
| 650 | 25,28 | 16,67 | 20,4 | 24,59 | 28,49 | 37,6 | 24,63 | 28,44 | 33,53 | 42,34 | 50,23 | 28,02 | 41,08 | 47,16 | 56,07 |
| 660 | 27,65 | 18,52 | 22,53 | 27,02 | 30,94 | 40,32 | 27,04 | 31,03 | 36,19 | 45,07 | 52,75 | 30,56 | 43,8 | 49,81 | 58,42 |
| 670 | 28,52 | 19,69 | 23,59 | 28,05 | 31,87 | 40,75 | 28,03 | 32 | 36,99 | 45,39 | 52,58 | 31,45 | 44,07 | 49,86 | 57,92 |
| 680 | 29,57 | 21,01 | 24,79 | 29,2 | 32,98 | 41,49 | 29,16 | 33,1 | 37,97 | 46,03 | 52,95 | 32,45 | 44,64 | 50,33 | 58,15 |
| 690 | 33,24 | 23,94 | 28,05 | 32,83 | 36,61 | 45,63 | 32,81 | 36,88 | 41,93 | 50,19 | 57,11 | 36,33 | 48,82 | 54,46 | 62,28 |
| 700 | 40,08 | 29,05 | 34,12 | 39,51 | 43,22 | 53,36 | 39,57 | 43,88 | 49,14 | 57,96 | 64,7 | 43,55 | 56,7 | 61,97 | 69,57 |

TABLEAU I

**Office européen**
**des brevets**

# RAPPORT DE RECHERCHE EUROPEENNE

Numéro de la demande

EP 03 29 3306

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 2002/172700 A1 (BEKELE HAIMANOT) 21 novembre 2002 (2002-11-21) * page 13, colonne 2, ligne 5-8; exemple 13 * --- | 1-18 | A61K7/021 |
| X | US 6 384 074 B1 (TSUCHIYA KATSUYOSHI ET AL) 7 mai 2002 (2002-05-07) * colonne 16, ligne 22-28; tableau 16 * --- | 1-18 | |
| X | US 5 804 203 A (HAHN GARY S ET AL) 8 septembre 1998 (1998-09-08) * colonne 4, ligne 52-54; exemple 9 * --- | 1-18 | |
| X | EP 1 002 515 A (SHISEIDO CO LTD) 24 mai 2000 (2000-05-24) * page 16, ligne 20; exemple 22 * ----- | 1-18 | |

**DOMAINES TECHNIQUES RECHERCHES (Int.Cl.7)**

A61K

Le présent rapport a été établi pour toutes les revendications

| Lieu de la recherche | Date d'achèvement de la recherche | Examinateur |
|---|---|---|
| MUNICH | 29 mars 2004 | Vayssié, S |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

 

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 03 29 3306

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-03-2004

| Document brevet cité au rapport de recherche | | Date de publication | Membre(s) de la famille de brevet(s) | | Date de publication |
|---|---|---|---|---|---|
| US 2002172700 | A1 | 21-11-2002 | CA | 2448240 A1 | 19-09-2002 |
| | | | EP | 1370234 A2 | 17-12-2003 |
| | | | WO | 02072055 A2 | 19-09-2002 |
| US 6384074 | B1 | 07-05-2002 | JP | 11302219 A | 02-11-1999 |
| | | | EP | 1252888 A1 | 30-10-2002 |
| | | | EP | 0970693 A1 | 12-01-2000 |
| | | | US | 6313214 B1 | 06-11-2001 |
| US 5804203 | A | 08-09-1998 | US | 5716625 A | 10-02-1998 |
| | | | AU | 3406997 A | 07-01-1998 |
| | | | CA | 2258726 A1 | 24-12-1997 |
| | | | EP | 0954274 A1 | 10-11-1999 |
| | | | WO | 9748371 A1 | 24-12-1997 |
| | | | US | 2003031727 A1 | 13-02-2003 |
| | | | AT | 222744 T | 15-09-2002 |
| | | | AU | 717182 B2 | 16-03-2000 |
| | | | AU | 4689796 A | 10-07-1996 |
| | | | BR | 9510529 A | 15-12-1998 |
| | | | CA | 2208246 A1 | 27-06-1996 |
| | | | CN | 1175206 A | 04-03-1998 |
| | | | CZ | 9701914 A3 | 12-11-1997 |
| | | | DE | 69527987 D1 | 02-10-2002 |
| | | | DE | 69527987 T2 | 28-05-2003 |
| | | | DK | 806933 T3 | 06-01-2003 |
| | | | EP | 1136065 A1 | 26-09-2001 |
| | | | EP | 0806933 A1 | 19-11-1997 |
| | | | ES | 2181812 T3 | 01-03-2003 |
| | | | FI | 972580 A | 19-08-1997 |
| | | | HK | 1004663 A1 | 21-03-2003 |
| | | | HU | 77099 A2 | 02-03-1998 |
| | | | JP | 11502505 T | 02-03-1999 |
| | | | NO | 972922 A | 21-08-1997 |
| | | | NZ | 301463 A | 27-04-2001 |
| | | | PL | 320935 A1 | 10-11-1997 |
| | | | PT | 806933 T | 31-01-2003 |
| | | | WO | 9619184 A1 | 27-06-1996 |
| | | | US | 2003124202 A1 | 03-07-2003 |
| | | | US | 6139850 A | 31-10-2000 |
| EP 1002515 | A | 24-05-2000 | EP | 1002515 A1 | 24-05-2000 |
| | | | CN | 1272782 T | 08-11-2000 |
| | | | WO | 9963950 A1 | 16-12-1999 |
| | | | JP | 2000053529 A | 22-02-2000 |

EPO FORM P0460

Pour tout renseignement concernant cette annexe : voir Journal Officiel de l'Office européen des brevets, No.12/82